# EUROPÄISCHE PATENTANMELDUNG

(11) **EP 1 136 502 A1**
(43) Veröffentlichungstag der Anmeldung: **26.09.2001**
(21) Anmeldenummer: 01104577.0
(22) Anmeldetag: 06.03.2001
(51) Int. Cl.: C07K 14/435, C12N 15/12, C12N 15/70, C12N 15/85, C07K 16/18, A01N 25/00, C12N 1/21, C12N 5/10, A01K 67/033

(54) **Ligandengesteuerte Anionenkanäle von Insekten**

(30) Priorität: 18.03.2000 DE 10013619
(71) Anmelder: Bayer Aktiengesellschaft, 51368 Leverkusen (DE)
(72) Erfinder: Franken, Eva-Maria, Dr., 42799 Leichlingen (DE); Friedrich, Gabi, Dr., 51377 Leverkusen (DE); Raming, Klaus, Dr., Redwood City, CA 94061 (US)

(57) **Zusammenfassung**

Die Erfindung betrifft Polypeptide, die neue Untereinheiten ligandengesteuerter Anionenkanäle von Insekten darstellen, sowie Nukleinsäuren, die für diese Polypeptide codieren und insbesondere deren Verwendung zum Auffinden von Wirkstoffen für den Pflanzenschutz.

## Beschreibung

Die Erfindung betrifft Polypeptide, die neue Untereinheiten ligandengesteuerter Anionenkanäle von Insekten darstellen, sowie Nukleinsäuren, die für diese Polypeptide codieren und insbesondere deren Verwendung zum Auffinden von Wirkstoffen für den Pflanzenschutz.

Ligandengesteuerte Anionenkanäle, wie z.B. der chloridabhängige GABA- oder der Glutamat-Rezeptor, spielen eine wichtige Rolle bei der Neurotransmission im Tierreich. Die Bindung des Liganden an den Rezeptor verursacht eine vorübergehende Öffnung des Kanals und gestattet den Durchstrom von Anionen. Man nimmt an, dass ein Rezeptor aus fünf Untereinheiten besteht, die sich um eine Pore gruppieren. Jede dieser Untereinheiten ist ein Protein, das aus einem extrazellulären N-terminalen Teil besteht, gefolgt von drei Transmembranregionen, einem intrazellulären Teil, sowie einer vierten Transmembranregion und einem kurzen extrazellulären C-terminalen Teil (Hosie et al., 1997).

In Insekten sind GABA und Glutamat die wichtigsten inhibitorischen Neurotransmitter des zentralen Nervensystems (Cleland, 1996; Delgado et al., 1989). Dementsprechend lassen sich GABA- und Glutamat-Rezeptoren an Präparaten zentraler Ganglien aus Insekten elektrophysiologisch nachweisen. Diese Rezeptoren sind außerdem der molekulare Angriffspunkt wichtiger natürlicher und synthetischer Insektizide (Sattelle, 1990, 1992; Cully et al., 1994, 1996; Arena et al., 1995), die als Agonisten (z.B. Avermectin) und Antagonisten (z.B. Fipronil) von ligandengesteuerten Anionenkanälen wirken. Zusätzlich unterstreicht das Fehlen des chloridabhängigen Glutamat-Rezeptors in höheren Tieren (Wirbeltieren) seine hohe Bedeutung als selektives Target.

Die Proteinsequenz einer Anzahl von chloridabhängigen GABA- und Glutamat-Rezeptoren von Insekten ist bereits bekannt. So sind z.B. in Drosophila melanogaster die Sequenzen von drei verschiedenen GABA-Rezeptor-Untereinheiten sowie von einer Glutamat-Rezeptor Untereinheit beschrieben (ffrench-Constant et al., 1991; Harvey et al., 1994; Henderson et al., 1993; Cully et al., 1996).

Es ist von großer praktischer Bedeutung, beispielsweise für die Suche nach neuen Insektiziden, neue Untereinheiten von ligandengesteuerten Anionkanälen aus Insekten bereitzustellen, wobei besonders solche von Interesse sind, die sich von den bekannten stärker unterscheiden, als dies zwischen funktionellen Homologen der Fall ist.

Der vorliegenden Erfindung liegt somit insbesondere die Aufgabe zugrunde, Untereinheiten ligandengesteuerter Anionenkanäle von Insekten und darauf aufbauende Testsysteme mit einem hohen Durchsatz an Testverbindungen (High Throughput Screening Assays; HTS-Assays) bereitzustellen.

Die Aufgabe wird gelöst durch die Bereitstellung von Polypeptiden, welche am Aufbau eines ligandengesteuerten Anionenkanals beteiligt sind und eine Aminosäuresequenz umfassen, die eine zumindest 70 %ige Identität, vorzugsweise eine zumindest 80 %ige Identität, besonders bevorzugt eine zumindest 90 %ige Identität, ganz besonders bevorzugt eine zumindest 95 %ige Identität, mit einer Sequenz gemäß SEQ ID NO: 2, 4, 6, 8, 10, 12, 14, 16 oder 18 über eine Länge von wenigstens 20, vorzugsweise wenigstens 25, besonders bevorzugt wenigstens 30 fortlaufenden Aminosäuren und ganz besonders bevorzugt über deren Gesamtlängen aufweisen.

Der Grad der Identität der Aminosäuresequenzen wird vorzugsweise bestimmt mit Hilfe des Programms GAP aus dem Programmpaket GCG, Version 9.1 unter Standardeinstellungen (Devereux et al., 1984).

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise an dem Peptid-Rückgrat, an der Aminosäure-Seitenkette, am Amino- und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylie-rungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen.

Ein funktioneller ligandengesteuerter Anionenkanal wird vorzugsweise durch eine Homo- oder Heteromerisierung der erfindungsgemäßen Polypeptide erreicht.

Die erfindungsgemäßen Polypeptide müssen nicht die Länge natürlich vorkommender Anionenkanal-Untereinheiten aufweisen, sondern können auch nur Fragmente davon sein, solange sie noch funktionelle ligandengesteuerte Anionenkanäle bilden können. Anionenkanäle, die im Vergleich zu Kanälen, die aus den erfindungsgemäßen Polypeptiden mit einer Aminosäuresequenz gemäß SEQ ID NO: 2, 4, 6, 8, 10, 12, 14, 16 oder 18 bestehen, eine um 50% höhere oder verminderte Aktivität ausüben, werden noch als erfindungsgemäß betrachtet. Dabei müssen die erfindungsgemäßen Polypeptide nicht von ligandengesteuerten Anionenkanälen aus Drosophila melanogaster ableitbar sein. Als erfindungsgemäß werden auch Polypeptide betrachtet, die ligandengesteuerte Anionenkanäle beispielsweise der folgenden Invertebraten entsprechen oder Fragmenten davon, die noch die biologische Aktivität dieser Kanäle ausüben können: Insekten, Nematoden, Arthropoden, Mollusken.

Die erfindungsgemäßen Polypeptide können im Vergleich zu der entsprechenden Region natürlich vorkommender ligandengesteuerter Anionenkanäle Deletionen oder Aminosäuresubstitutionen aufweisen, solange sie noch funktionelle Kanäle bilden können. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Der Ausdruck "funktioneller ligandengesteuerter Anionenkanal" bzw. "funktioneller Kanal", wie er hierin verwendet wird, bezieht sich auf einen Anionenkanal, der nach Bindung eines Liganden für Anionen durchlässig wird.

Gegenstand der vorliegenden Erfindung sind weiterhin ligandengesteuerte Anionenkanäle, die aus den erfindungsgemäßen Polypeptiden aufgebaut sind.

Bevorzugte Ausführungsformen der erfindungsgemäßen Polypeptide stellen solche Polypeptide dar, die am Aufbau ligandengesteuerter Anionenkanäle von Drosophila melanogaster beteiligt sind und eine Aminosäuresequenz gemäß SEQ ID NO: 2, 4, 6, 8, 10, 12, 14, 16 oder 18 besitzen.

Gegenstand der vorliegenden Erfindung sind auch Nukleinsäuren, die für die erfindungsgemäßen Polypeptide codieren.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Bevorzugte Ausführungsformen der erfindungsgemäßen Nukleinsäuren stellen cDNAs dar, welche eine Nukleotidsequenz gemäß SEQ ID NO: 1, 3, 5, 7, 9, 11, 13, 15 oder 17 besitzen.

Nukleinsäuren, welche unter stringenten Bedingungen an die Sequenzen gemäß SEQ ID NO: 1, 3, 5, 7, 9, 11, 13, 15 oder 17 hybridisieren, sind ebenfalls von der vorliegenden Erfindung umfasst.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können ausgehend von der hierin offenbarten Sequenzinformation beispielsweise DNA-Fragmente aus anderen Insekten als Drosophila melanogaster isoliert werden, welche für Untereinheiten ligandengesteuerter Anionenkanäle codieren.

Bevorzugte Hybridisierungsbedingungen sind nachstehend angegeben:

Hybridisierungslösung: 6X SSC / 0 % Formamid, bevorzugte Hybridisierungslösung: 6X SSC / 25 % Formamid

Hybridisierungstemperatur: 34°C, bevorzugte Hybridisierungstemperatur: 42°C
1. Waschschritt: 2X SSC bei 40°C,
2. Waschschritt: 2X SSC bei 45°C; bevorzugter 2. Waschschritt: 0,6X SSC bei 55°C; besonders bevorzugter 2. Waschschritt: 0,3X SSC bei 65°C.

Weiterhin sind von der vorliegenden Erfindung Nukleinsäuren umfasst, die eine zumindest 70 %ige Identität, vorzugsweise eine zumindest 80 %ige Identität, besonders bevorzugt eine zumindest 90 %ige Identität, ganz besonders bevorzugt eine zumindest 95 %ige Identität, mit einer Sequenz gemäß SEQ ID NO: 1, 3, 5, 7, 9, 11, 13, 15 oder 17 über eine Länge von wenigstens 20, vorzugsweise wenigstens 25, besonders bevorzugt wenigstens 30 fortlaufenden Nukleotiden und ganz besonders bevorzugt über deren Gesamtlängen aufweisen.

Der Grad der Identität der Nukleinsäuresequenzen wird vorzugsweise bestimmt mit Hilfe des Programms GAP aus dem Programmpaket GCG, Version 9.1 unter Standardeinstellungen.

Die Sequenzen gemäß Genbank-Zugangsnummern (Acc No.) AC002502, AF145639 und AC004420 sind durch Bezugnahme Bestandteil der vorliegenden Beschreibung.

Gegenstand der vorliegenden Erfindung sind weiterhin DNA-Konstrukte, die eine erfindungsgemäße Nukleinsäure und einen heterologen Promotor umfassen.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert. Der Ausdruck "Promotor", wie er hierin verwendet wird, bezieht sich allgemein auf Expressionskontrollsequenzen.

Die Auswahl von heterologen Promotoren ist davon abhängig, ob zur Expression pro- oder eukaryotische Zellen oder zellfreie Systeme verwendet werden. Beispiele für heterologe Promotoren sind der frühe oder späte Promotor des SV40, des Adenovirus oder des Cytomegalovirus, das lac-System, das trp-System, die Haupt-Operator- und Promotorregionen des Phagen lambda, die Kontrollregionen des fd-Hüllproteins, der Promotor der 3-Phosphoglyceratkinase, der Promotor der Sauren Phosphatase und der Promotor des α-Mating-Faktors der Hefe.

Gegenstand der Erfindung sind weiterhin Vektoren, die eine erfindungsgemäße Nukleinsäure bzw. ein erfindungsgemäßes DNA-Konstrukt enthalten. Als Vektoren können alle in molekularbiologischen Laboratorien verwendete Plasmide, Phasmide, Cosmide, YACs oder künstliche Chromosomen verwendet werden.

Gegenstand der vorliegenden Erfindung sind auch Wirtszellen, die eine erfindungsgemäße Nukleinsäure, ein erfindungsgemäßes DNA-Konstrukt oder einen erfindungsgemäßen Vektor enthalten.

Der Ausdruck "Wirtszelle", wie er hierin verwendet wird, bezieht sich auf Zellen, die natürlicherweise die erfindungsgemäßen Nukleinsäuren nicht enthalten.

Als Wirtszellen eignen sich sowohl prokaryotische Zellen, wie Bakterien der Gattungen Bacillus, Pseudomonas, Streptomyces, Streptococcus, Staphylococcus, vorzugsweise E. coli, als auch eukaryotische Zellen, wie Hefen, Säuger-, Amphibien-, Insekten- oder Pflanzenzellen. Bevorzugte eukaryotische Wirtszellen sind HEK-293-, Schneider S2-, Spodoptera Sf9-, Kc-, CHO-, COS1-, COS7-, HeLa-, C127-, 3T3- oder BHK-Zellen und insbesondere Xenopus-Oocyten.

Weiterhin sind Antikörper Gegenstand der Erfindung, die spezifisch an die vorstehend genannten Polypeptide bzw. Kanäle binden. Die Herstellung solcher Antikörper erfolgt auf die übliche Weise. Beispielsweise können solche Antikörper produziert werden durch die Injektion eines substantiell immunkompetenten Wirts mit einer für die Antikörperproduktion effektiven Menge eines erfindungsgemäßen Polypeptids oder eines Fragments davon und durch nachfolgende Gewinnung dieses Antikörpers. Weiterhin läßt sich in an sich bekannter Weise eine immortalisierte Zellinie erhalten, die monoklonale Antikörper produziert. Die Antikörper können gegebenenfalls mit einem Nachweisreagenz markiert sein. Bevorzugte Beispiele für ein solches Nachweis-Reagenz sind Enzyme, radioaktiv markierte Elemente, fluoreszierende Chemikalien oder Biotin. Anstelle des vollständigen Antikörpers können auch Fragmente eingesetzt werden, die die gewünschten spezifischen Bindungseigenschaften besitzen. Daher erstreckt sich der Ausdruck "Antikörper", wie er hierin verwendet wird, auch auf Teile vollständiger Antikörper, wie Fa-, F(ab')₂- oder Fv-Fragmente, welche noch die Fähigkeit besitzen, an die Epitope der erfindungsgemäßen Polypeptide zu binden.

Die erfindungsgemäßen Nukleinsäuren können insbesondere zur Herstellung transgener Invertebraten verwendet werden. Diese können in Testsysteme eingesetzt werden, die auf einer vom Wildtyp abweichenden Expression der erfindungsgemäßen Polypeptide basieren. Ferner kann man auf der Grundlage der hierin offenbarten Informationen transgene Invertebraten herstellen, bei denen durch die Modifikation anderer Gene oder Promotoren eine Veränderung der Expression der erfindungsgemäßen Polypeptide eintritt.

Die Herstellung der transgenen Invertebraten erfolgt beispielsweise bei Drosophila melanogaster durch P-Element vermittelten Gentransfer (Hay et al., 1997) oder in Caenorhabditis elegans durch Transposon-vermittelten Gentransfer (z.B. durch Tc1; Plasterk, 1996).

Gegenstand der Erfindung sind somit auch transgene Invertebraten, die zumindest eine der erfindungsgemäßen Nukleinsäuren enthalten, vorzugsweise transgene Invertebraten der Arten Drosophila melanogaster oder Caenorhabditis elegans, sowie deren transgene Nachkommen. Vorzugsweise enthalten die transgenen Invertebraten die erfindungsgemäßen Polypeptide in einer vom Wildtyp abweichenden Form .

Gegenstand der vorliegenden Erfindung sind weiterhin Verfahren zum Herstellen der erfindungsgemäßen Polypeptide. Zur Herstellung der Polypeptide, die von den erfindungsgemäßen Nukleinsäuren codiert werden, können Wirtszellen, die eine der erfindungsgemäßen Nukleinsäuren enthalten, unter geeigneten Bedingungen kultiviert werden. Dabei kann die zu exprimierende Nukleinsäure an die "Codon Usage" der Wirtszellen angepaßt werden. Die gewünschten Polypeptide können danach auf übliche Weise aus den Zellen oder dem Kulturmedium isoliert werden. Die Polypeptide können auch in *in vitro*-Systemen hergestellt werden.

Ein schnelles Verfahren zum Isolieren der erfindungsgemäßen Polypeptide, die von Wirtszellen unter Verwendung einer erfindungsgemäßen Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Glutathion S-Transferase sein. Das Fusionsprotein kann dann an einer Glutathion-Affinitätssäule gereinigt werden. Der Fusionspartner kann durch partielle proteolytische Spaltung beispielsweise an Linkem zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren auf präparativer Elektrophorese, FPLC, HPLC (z.B. unter Anwendung von Gelfiltrations-, Reversphasen- oder leicht hydrophoben Säulen), Gelfiltration, differentielle Präzipitation, Ionenaustausch-Chromatographie und Affinitätschromatographie.

Da ligandengesteuerte Anionenkanäle Membranproteine darstellen, werden in den Reinigungsverfahren vorzugsweise Detergensextraktionen durchgeführt, beispielsweise unter Verwendung von Detergenzien, die die Sekundär- und Tertiärstrukturen der Polypeptide nicht oder nur wenig beeinflussen, wie nicht-ionische Detergenzien.

Die Reinigung der erfindungsgemäßen Polypeptide kann die Isolierung von Membranen ausgehend von Wirtszellen, die die erfindungsgemäßen Nukleinsäuren exprimieren, umfassen. Vorzugsweise exprimieren solche Zellen die erfindungsgemäßen Polypeptide in einer ausreichenden Kopienanzahl, so dass die Menge der Polypeptide in einer Membranfraktion mindestens 10-fach höher ist als diejenige, die in vergleichbaren Membranen von Zellen gefunden wird, die ligandengesteuerte Anionenkanäle natürlicherweise exprimieren; besonders bevorzugt ist Menge mindestens 100-fach, ganz besonders bevorzugt mindestens 1000-fach höher.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Ferner sind auch Verfahren zum Herstellen der erfindungsgemäßen Nukleinsäuren Gegenstand der vorliegenden Erfindung. Die erfindungsgemäßen Nukleinsäuren können auf die übliche Weise hergestellt werden. Beispielsweise können die Nukleinsäuremoleküle vollständig chemisch synthetisiert werden. Man kann auch nur kurze Stücke der erfindungsgemäßen Sequenzen chemisch synthetisieren und solche Oligonukleotide radioaktiv oder mit einem Fluoreszenzfarbstoff markieren. Die markierten Oligonukleotide können verwendet werden, um ausgehend von Insekten-mRNA hergestellte cDNA-Banken oder ausgehend von genomischer Insekten-DNA hergestellte genomische Banken zu durchsuchen. Klone, an die die markierten Oligonukleotide hybridisieren, werden zur Isolierung der betreffenden DNA ausgewählt. Nach der Charakterisierung der isolierten DNA erhält man auf einfache Weise die erfindungsgemäßen Nukleinsäuren.

Die erfindungsgemäßen Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bedeutet DNA-Moleküle, die aus 10 bis 50 Nukleotiden, vorzugsweise 15 bis 30 Nukleotiden, bestehen. Sie werden chemisch synthetisiert und können als Sonden verwendet werden.

Mit Hilfe der erfindungsgemäßen Nukleinsäuren können neue Wirkstoffe für den Pflanzenschutz bzw. pharmazeutische Wirkstoffe für die Behandlung von Mensch und Tier, wie chemische Verbindungen, welche als Modulatoren, insbesondere als Agonisten oder Antagonisten, die Eigenschaften der erfindungsgemäßen ligandengesteuerten Anionenkanäle verändern, identifiziert werden. Dazu wird ein rekombinantes DNA-Molekül, das zumindest eine erfindungsgemäße Nukleinsäure umfasst, in eine geeignete Wirtszelle eingebracht. Die Wirtzelle wird in Gegenwart einer Verbindung oder einer Probe, welche eine Vielzahl von Verbindungen umfasst, unter Bedingungen kultiviert, die die Expression der erfindungsgemäßen Rezeptoren erlauben. Eine Veränderung der Rezeptoreigenschaften kann - wie nachstehend in Beispiel 2-beschrieben - detektiert werden. Auf diese Weise ist es möglich, beispielsweise insektizide Substanzen aufzufinden.

Ein funktioneller ligandengesteuerter Anionenkanal kann durch funktionelle oder Bindungsassays nachgewiesen werden (Rudy et al., 1992). Ein funktioneller Nachweis ist z.B. die elektrophysiologische Ableitung von Zellen oder Oocyten, die ligandengesteuerte Anionenkanäle exprimieren und auf Zugabe von Liganden mit einem Anionenflux reagieren (führt zu Potentialänderung an der Zellmembran). Typischerweise sind die liganden-induzierten Ströme abhängig von der Konzentration des applizierten Liganden. Neben der elektrophysiologischen Ableitung können Potentialänderungen auch durch z.B. potentiometrische Farbstoffe visualisiert werden. In einem typischen Bindungsassay werden potentielle Agonisten und Antagonisten durch ihre Fähigkeit identifiziert, bekannte radioaktiv markierte Liganden vom Kanal zu verdrängen. Geeignete bekannte Liganden sind z.B. GABA, Glutamat, Glycin, Muscimol, EBOB (n-Propyletynylbicycloorthobenzoat), BIDN (3,3-bis (trifluormethyl) bicyclo [2,2,1] heptan-2,2-dicarbonitril), Avermectin, Fipronil.

Solche Assays können im Rahmen eines High Throughput Screenings (HTS) zur Auffindung von Agonisten, Antagonisten, Modulatoren verwendet werden.

Der Ausdruck "Agonist", wie er hierin verwendet wird, bezieht sich auf ein Molekül, das ligandengesteuerte Anionenkanäle aktiviert.

Der Ausdruck "Antagonist", wie er hierin verwendet wird, bezieht sich auf ein Molekül, das einen Agonisten von seiner Bindungsstelle verdrängt.

Der Ausdruck "Modulator", wie er hierin verwendet wird, stellt den Oberbegriff zu Agonist bzw. Antagonist dar. Modulatoren können kleine organisch-chemische Moleküle, Peptide oder Antikörper sein, die an die erfindungsgemäßen Polypeptide binden. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können Mimetika von natürlichen Substraten und Liganden darstellen.

Vorzugsweise handelt es sich bei den Modulatoren um kleine organisch-chemische Verbindungen.

Die Bindung der Modulatoren an die erfindungsgemäßen Polypeptide kann die zellulären Vorgänge auf eine Weise verändern, die zum Absterben der damit behandelten Insekten führt.

Daher erstreckt sich die vorliegende Erfindung auch auf die Verwendung von Modulatoren der erfindungsgemäßen Polypeptide bzw. der erfindungsgemäßen ligandengesteuerten Anionenkanäle als Insektizide.

Die erfindungsgemäßen Nukleinsäuren ermöglichen auch das Auffinden von Verbindungen, die an die erfindungsgemäßen Kanäle binden. Diese können ebenfalls als Insektizide auf Pflanzen angewandt werden. Beispielsweise werden Wirtszellen, die die erfindungsgemäßen Nukleinsäuren enthalten und die entsprechenden Kanäle bzw. Polypeptide exprimieren oder die Genprodukte selbst mit einer Verbindung oder einem Gemisch von Verbindungen unter Bedingungen in Kontakt gebracht, die die Wechselwirkung zumindest einer Verbindung mit den Wirtszellen, den Kanälen oder den einzelnen Polypeptiden erlauben.

Unter Verwendung von Wirtszellen oder transgenen Invertebraten, die die erfindungsgemäßen Nukleinsäuren enthalten, ist es auch möglich, Substanzen aufzufinden, welche die Expression der Kanäle verändern.

Die vorstehend beschriebenen erfindungsgemäßen Nukleinsäuren, Vektoren und regulatorischen Regionen können außerdem zum Auffinden von Genen verwendet werden, die für Polypeptide codieren, welche am Aufbau von funktionell ähnlichen ligandengesteuerten Anionenkanälen in Insekten beteiligt sind. Unter funktionell ähnlichen Rezeptoren werden gemäß der vorliegenden Erfindung Kanäle verstanden, die Polypeptide umfassen, welche sich zwar hinsichtlich der Aminosäuresequenz von den hierin beschriebenen Polypeptiden unterscheiden, aber im wesentlichen dieselben Funktionen haben.

### Erläuterungen zum Sequenzprotokoll und zu den Abbildungen:

SEQ ID NO: 1, 3, 5, 7, 9, 11, 13, 15 und 17 zeigen die Nukleotid- und Aminosäuresequenzen der isolierten ligandengesteuerten Anionenkanal-cDNAs. SEQ ID NO: 2, 4, 6, 8, 10, 12, 14, 16 und 18 zeigen ferner die Aminosäuresequenzen der von den ligandengesteuerten Anionenkanal-cDNA-Sequenzen abgeleiteten Polypeptide.

### Beispiele:

### Beispiel 1

### Isolierung der beschriebenen Polynukleotide

Die Manipulation von Polynukleotiden erfolgte nach Standardmethoden der rekombinanten DNA-Technologie (Sambrook et al., 1989). Die bioinformatische Bearbeitung von Nukleotid- und Proteinsequenzen erfolgten mit dem Programmpaket GCG Version 9.1 (GCG Genetics Computer Group, Inc., Madison Wisconsin, USA).

### Beispiel 2

### Generierung der Expressionskonstrukte

Mittels Polymerasekettenreaktion (PCR) wurden die Sequenzbereiche der SEQ ID NO: 1, 3, 5, 7, 9, 11, 13, 15 und 17 amplifiziert und in die Vektoren pcDNA3.1/Neo (Invitrogen, Groningen) und pIE1-3/4 (Novagen, Madison WI, USA) einkloniert.

### Heterologe Expression

Die funktionelle Expression der ligandengesteuerten Anionenkanäle aus Insekten erfolgte in Sf9-Zellinien und Xenopus-Oocyten. Dazu wurden Expressionsvektoren mit den unterschiedliche Untereinheiten einzeln und in verschiedenen Kombinationen in die Zellen / Oozyten eingebracht.

### Messungen in Sf9-Zellen

### 1. Zellkultur

Sf9-Zellen wurden in 50 ml CELLSTAR Zellkulturflaschen (Greiner, Nürtingen, Deutschland) bei 26°C in 5 ml SF-900 II Zellkulturmedium mit L-Glutamin (Gibco BRL, Karlsruhe, Deutschland) kultiviert.

### 2. Transfektion

Zur Transfektion wurden je 1 x 10⁵ Zellen pro Loch einer 4-Loch NUNCLON Zellkulturschale (Nunc, Wiesbaden, Deutschland) ausgesät. Die Transfektion erfolgte nach Standardprotokollen mit FuGENE 6 (Roche Molecular Biochemicals, Mannheim, Deutschland). Pro Expressionskonstrukt wurde 1 µg Vektor-DNS verwendet. Die elektrophysiologische Messung erfolgte 26 bis 48 h nach der Transfektion.

### Oocyten-Messungen

### 1. Präparation der Oocyten

Die Oocyten stammen von einem adulten weiblichen Xenopus laevis-Frosch (Firma Horst Kähler, Hamburg, Deutschland). Die Frösche wurden in großen Tanks mit zirkulierendem Wasser bei einer Wassertemperatur von 20 - 24°C gehalten. Teile des Frosch-Ovars wurden unter vollständiger Anaesthesie durch einen kleinen Schnitt im Abdomen (ca. 1cm) entnommen. Anschließend wurde das Ovar unter ständigem Schütteln für ca. 140min in 25ml Kollagenase (Typ I, C-0130, SIGMA-ALDRICH CHEMIE GmbH, Deisenhofen, Deutschland; 355U/ml, angesetzt in Barth's Lösung ohne Calcium in mM: NaCl 88, KCl 1, MgSO4 0.82, NaHCO3 2.4, Tris/HCl 5, pH7.4) behandelt. Die Oocyten wurden dann mit Barth's Lösung ohne Calcium gewaschen. Nur Oocyten im Reifestadium V (Dumont, 1972) wurden für die weitere Behandlung ausgewählt und in Mikrotiterplatten (Nunc Micro Well™ Platten, Kat. Nr. 245128 + 263339 (Deckel), Nunc GmbH & Co. KG, Wiesbaden, Deutschland), gefüllt mit Barth's-Lösung (in mM: NaCl 88, KCl 1, MgSO₄ 0.82, Ca(NO₃)₂ 0.33, CaCl₂ 0.41, NaHCO₃ 2.4, Tris/HCl 5, pH7.4) sowie Gentamicin (Gentamicin Sulfate, G-3632, SIGMA-ALDRICH CHEMIE GmbH, Deisenhofen, Deutschland; 100U/ml), überführt. Die Oocyten wurden dann bei 19.2°C in einem Kühl-Brutschrank (Typ KB 53, WTB Binder Labortechnik GmbH, Tuttlingen, Deutschland) aufbewahrt.

### 2. Injektion der Oocyten

Injektionselektroden, mit einem Durchmesser von 10 - 15 µm, wurden mit einem Pipetten-Puller (Typ L/M-3P-A, List-electronic, Darmstadt-Eberstadt, Deutschland) hergestellt. Vor der Injektion wurden Aliquots mit der DNA aufgetaut und mit Wasser auf eine Endkonzentration von 10ng/µl verdünnt. Die DNA-Proben wurden mit 3200g für 120s zentrifugiert (Typ Biofuge 13, Heraeus Instruments GmbH, Hanau, Deutschland). Anschließend wurde ein ausgezogener PE-Schlauch als Transferschlauch benutzt, um die Pipetten von hinten zu befüllen. Die Injektionselektroden wurden an einer X,Y,Z-Verfahreinheit (Bearbeitungszentrum EP1090, isel-automation, Eiterfeld, Deutschland) befestigt. Mit Hilfe eines Macintosh Computer wurde die Oocyten in den Vertiefungen der Mikrotiterplatten angefahren und durch kurze Druckapplikation (0.5-3.0bar, 3-6s) ca. 50nl der DNA-Lösung in die Oocyte injiziert.

### 3. Elektrophysiologische Messungen

Für die elektrophysiologischen Messungen wurde eine Zwei-Elektroden-Spannungsklemme mit einem TURBO TEC-10CD (npi electronic GmbH, Tamm, Deutschland) Verstärker durchgeführt. Die hierfür notwendigen Mikropipetten wurden aus Aluminiumsilikatglas (Kapillarrohr, Art.-Nr. 14 630 29, 1=100mm, Øa=1,60mm, Øi=1,22mm, Hilgenberg GmbH, Malsfeld, Deutschland) in zwei Zügen gezogen (Hamill et al., 1981). Strom- und Spannungselektroden hatten einen Durchmesser von 1 - 3µm und wurden mit 1.5M KCl und 1.5M Kaliumacteat gefüllt. Die Pipetten hatten einen kapazitiven Widerstand von 0.2 - 0.5 MW. Für die elektrophysiologischen Messungen wurden die Oocyten in eine kleine Kammer überführt, die kontinuierlich mit normaler Rimland-Lösung (in mM: KCl 90, MgCl₂ 3, HEPES 5, pH 7.2) gespült wurde. Für eine Substanzapplikation wurde die Perfusionslösung durch eine Substanzlösung mit gleicher Zusammensetzung und zusätzlich der gewünschten Substanzkonzentration ausgetauscht. Für die Überprüfung der Expression der DNA wurde das Haltepotential (-60 mV) der Membran nacheinander sprunghaft auf 10 depolarisierende und 10 hyperporalisierende Potentiale geklemmt. Die Dauer der Pulse betrug 100 ms. Das Spannungsinterval betrug pro Sprung 10 mV. Nicht reagierende Oocyten wurden verworfen. Alle weiteren wurden für die Substanztestung verwendet. Die Daten wurden mittels eines YT-Schreibers (YT- Schreiber, Model BD 111, Kipp & Zonen Delft BV, AM Delft, Niederlande) dokumentiert. Wenn Testsubstanzen in Konzentrationsreihen untersucht wurden, sind diese Messungen an mindestens zwei verschiedenen Oocyten und an mindestens fünf verschiedenen Konzentrationen durchgeführt worden. Die Substanzen sind direkt und ohne Vorinkubation auf ihren Antagonismus geprüft worden. Die einzelnen Werte wurden in Origin (Auswertesoftware Microcal Origin, Microcal Software, Inc., Northampton, MA 01060-4410 USA (Additive GmbH, Friedrichsdorf/Ts, Deutschland) eingegeben. Mittelwerte und Standardabweichung wurden mit Origin berechnet. Diese Messungen wurden mindestens zweimal durchgeführt.

### Literatur:

Arena J.P. et al. (1995), The mechanism of action of avermectins in Caenorhabditis elegans: correlation between activation of glutamate-sensitive chloride current, membrane binding, and biological activity, J. Parasitol. 81 (2), 286-294

Cleland T.A. (1996), Inhibitory glutamate receptor channels, Mol. Neurobiol. 13 (2), 97-136

Cully D.F. et al. (1994), Cloning of an avermectin-sensitive glutamate-gated chloride channel from Caenorhabditis elegans, Nature 371, 707-711

Cully D.F. et al. (1996), Identification of a Drosophila melanogaster glutamate-gated chloride channel sensitive to the antiparasitic agent avermectin, J. Biol. Chem. 271 (33), 20187-20191

Delgado R. (1989), L-glutamate activates excitatory and inhibitory channels in Drosophila larval muscle, FEBS letters 243 (2), 337-342

Devereux et al. (1984), Nucleic Acids Research 12, 387

Dumont, J.N. (1972), Oogenesis in *Xenopus laevis* (Daudin). 1. Stages of oocyte development in laboratory maintained animals, J. Morphol. 136: 153-180

ffrench-Constant R.H. et al. (1991), Proc. Natl. Acad. Sci. U.S.A.88, 7209-7213

Harvey R.J. et al. (1994), Sequence of a Drosophila ligand-gated ion-channel polypeptide with an unusual amino-terminal extracellular domain, J. Neurochem. 62, 2480-2483

Hay et al. (1997), P element insertion-dependent gene activation in the Drosophila eye, Proceedings of The National Academy of Sciences of The United States of America 94 (10), 5195-5200

Henderson J.E. et al. (1993), Characterization of a putative gamma-aminobutyric acid (GABA) receptor beta subunit gene from Drosophila melanogaster, Biochem. Biophys. Res. Commun. 193, 474-482

Hosie A.M. et al. (1997), Molecular biology of insect neuronal GABA receptors, TINS 20, 578-583

Plasterk (1996), The Tc1/mariner transposon family, Transposable Elements/Current Topics in Microbiology and Immunology 204, 125-143

Rudy et al., eds. (1992), Methods in Enzymology 207, Academic Press, Inc., San Diego, CA

Sambrook et al. (1989), Molecular Cloning, A Laboratory Manual, 2nd ed. Cold Spring Harbour Press

Sattelle D.B. (1990), GABA Receptors of Insects, Advances in Insect Physiology 22, 1-113

Satelle D.B. (1992), Receptors for L-glutamate and GABA in the nervous system of an insect (Periplaneta americana), Comp. Biochem. Physiol. C. 103 (3), 429-438

## Patentansprüche

1. Polypeptid, welches am Aufbau eines ligandengesteuerten Anionenkanals beteiligt ist und eine Aminosäuresequenz umfasst, die eine zumindest 70%ige Identität mit einer Sequenz gemäß SEQ ID NO: 2, 4, 6, 8, 10, 12, 14, 16 oder 18 aufweist.

2. Polypeptid gemäß Anspruch 1, **dadurch gekennzeichnet, dass** die Aminosäuresequenz einer Sequenz gemäß SEQ ID NO: 2, 4, 6, 8, 10, 12, 14, 16 oder 18 entspricht.

3. Ligandengesteuerter Anionenkanal umfassend ein Polypeptid gemäß Anspruch 1 oder 2.

4. Nukleinsäure umfassend eine Nukleotidsequenz, die für ein Polypeptid gemäß Anspruch 1 oder 2 codiert.

5. Nukleinsäure gemäß Anspruch 4, **dadurch gekennzeichnet, dass** es sich um einzelsträngige oder doppelsträngige DNA oder RNA handelt.

6. Nukleinsäure gemäß Anspruch 5, **dadurch gekennzeichnet, dass** es sich um Fragmente genomischer DNA oder cDNA handelt.

7. Nukleinsäure gemäß Anspruch 4, **dadurch gekennzeichnet, dass** die Nukleotidsequenz einer Sequenz gemäß SEQ ID NO: 1, 3, 5, 7, 9, 11, 13, 15 oder 17 entspricht.

8. Nukleinsäure gemäß Anspruch 4, **dadurch gekennzeichnet, dass** sie unter stringenten Bedingungen an die Sequenzen gemäß SEQ ID NO: 1, 3, 5, 7, 9, 11, 13, 15 oder 17 hybridisiert.

9. DNA-Konstrukt umfassend eine Nukleinsäure gemäß einem der Ansprüche 4 bis 8 und einen heterologen Promotor.

10. Vektor umfassend eine Nukleinsäure gemäß einem der Ansprüche 4 bis 8 oder ein DNA-Konstrukt gemäß Anspruch 9.

11. Vektor gemäß nach Anspruch 10, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

12. Wirtszelle enthaltend eine Nukleinsäure gemäß einem der Ansprüche 4 bis 8, ein DNA-Konstrukt gemäß Anspruch 9 oder einen Vektor gemäß Anspruch 10 oder 11.

13. Wirtszelle gemäß Anspruch 12, **dadurch gekennzeichnet, dass** es sich um eine prokaryotische Zelle, insbesondere um E. coli, handelt.

14. Wirtszelle gemäß Anspruch 12, **dadurch gekennzeichnet, dass** es sich um eine eukaryotische Zelle, insbesondere um eine Säuger- oder Insektenzelle, handelt.

15. Antikörper, welcher spezifisch an ein Polypeptid gemäß Anspruch 1 oder 2, oder an einen ligandengesteuerten Anionenkanal gemäß Anspruch 3 bindet.

16. Transgener Invertebrat enthaltend eine Nukleinsäure gemäß einem der Ansprüche 4 bis 8.

17. Transgener Invertebrat nach Anspruch 16, **dadurch gekennzeichnet, dass** es sich um Drosophila melanogaster oder Caenorhabditis elegans handelt.

18. Transgene Nachkommen eines Invertebraten gemäß Anspruch 16 oder 17.

19. Verfahren zum Herstellen eines Polypeptids gemäß Anspruch 1 oder 2, umfassend
(a) das Kultivieren einer Wirtszelle gemäß einem der Ansprüche 12 bis 14 unter Bedingungen, die die Expression der Nukleinsäure gemäß einem der Ansprüch 4 bis 8 gewährleisten, oder
(b) das Exprimieren einer Nukleinsäure gemäß einem der Ansprüche 4 bis 8 in einem *in vitro*-System, und
(c) die Gewinnung des Polypeptids aus der Zelle, dem Kulturmedium oder dem *in vitro*-System.

20. Verfahren zum Herstellen einer Nukleinsäure gemäß einem der Ansprüche 4 bis 8, umfassend die folgenden Schritte:
(a) Vollständige chemische Synthese auf an sich bekannte Weise, oder
(b) chemische Synthese von Oligonukleotiden, Markieren der Oligonukleotide, Hybridisieren der Oligonukleotide an DNA einer genomischen oder cDNA-Bank, die ausgehend von genomischer DNA bzw. mRNA aus Insektenzellen hergestellt wurde, Selektieren von positiven Klonen und Isolieren der hybridisierenden DNA aus positiven Klonen, oder
(c) chemische Synthese von Oligonukleotiden und Amplifizierung der Ziel-DNA mittels PCR.

21. Verfahren zum Herstellen eines transgenen Invertebraten gemäß Anspruch 16 oder 17, umfassend das Einbringen einer Nukleinsäure gemäß einem der Ansprüche 4 bis 8 oder eines Vektors gemäß Anspruch 10 oder 11.

22. Verfahren zum Auffinden neuer Wirkstoffe für den Pflanzenschutz, insbesondere von Verbindungen, welche die Eigenschaften von Polypeptiden gemäß Anspruch 1 oder 2 verändern, umfassend die folgenden Schritte:
(a) Bereitstellen einer Wirtszelle gemäß einem der Ansprüche 12 bis 14,
(b) Kultivieren der Wirtszelle in der Gegenwart einer chemischen Verbindung oder eines Gemischs von chemischen Verbindungen, und
(c) Detektieren veränderter Eigenschaften.

23. Verfahren zum Auffinden einer chemischen Verbindung, die an ein Polypeptid gemäß Anspruch 1 oder 2, oder an einen ligandengesteuerten Anionenkanal gemäß Anspruch 3 bindet, umfassend die folgenden Schritte:
(a) Inkontaktbringen eines Polypeptids gemäß Anspruch 1 oder 2, oder eines ligandengesteuerten Anionenkanals gemäß Anspruch 3, oder einer Wirtszelle gemäß einem der Ansprüche 12 bis 14 mit einer chemischen Verbindung oder einem Gemisch von chemischen Verbindungen unter Bedingungen, die die Interaktion einer chemischen Verbindung mit dem Polypeptid erlauben, und
(b) Bestimmen der chemischen Verbindung, die spezifisch an das Polypeptid bindet.

24. Verfahren zum Auffinden einer chemischen Verbindung, die die Expression eines Polypeptids gemäß Anspruch 1 oder 2 verändert, umfassend die folgenden Schritte:
(a) Inkontaktbringen einer Wirtszelle gemäß einem der Ansprüche 12 bis 14 oder eines transgenen Invertebraten gemäß Anspruch 16 oder 17 mit einer chemischen Verbindung oder einem Gemisch von chemischen Verbindungen,
(b) Bestimmen der Konzentration des Polypeptids gemäß Anspruch 1 oder 2, und
(c) Bestimmen der chemischen Verbindung, die die Expression des Polypeptids spezifisch beeinflusst.

25. Verwendung eines Polypeptids gemäß Anspruch 1 oder 2, eines ligandengesteuerten Anionenkanals gemäß Anspruch 3, einer Nukleinsäure gemäß einem der Ansprüche 4 bis 8, eines Vektors gemäß Anspruch 10 oder 11, einer Wirtszelle gemäß einem der Ansprüche 12 bis 14, eines Antikörpers gemäß Anspruch 15 oder eines transgenen Invertebraten gemäß Anspruch 16 oder 17 zum Auffinden neuer Wirkstoffe für den Pflanzenschutz oder zum Auffinden von Genen, die für Polypeptide codieren, welche am Aufbau von funktionell ähnlichen ligandengesteuerte Anionenkanäle in Insekten beteiligt sind.

26. Verwendung eines Modulators eines Polypeptids gemäß Anspruch 1 oder 2, oder eines ligandengesteuerten Anionenkanals gemäß Anspruch 3 als Insektizid.


SEQUENZPROTOKOLL

<110> Bayer Aktiengesellschaft

<120> Ligandengesteuerte Anionenkanäle von Insekten

<130> Le A 34 397

<140>
<141>

<150> DE 100 136 19.2
<151> 2000-03-18

<160> 18

<170> PatentIn Ver. 2.1

<210> 1
<211> 1575
<212> DNA
<213> Drosophila melanogaster

<220>
<221> CDS
<222> (1)..(1572)

<400> 1
atg gag tgg tcg ctc atc ata tcg att ctg ctc aac tgc ctc tac gcc 48
Met Glu Trp Ser Leu Ile Ile Ser Ile Leu Leu Asn Cys Leu Tyr Ala
1               5                   10                  15

agc gcc tcc gcg ctc gac gag gat tgc ccc acc ttg gac gat ggg gac 96
Ser Ala Ser Ala Leu Asp Glu Asp Cys Pro Thr Leu Asp Asp Gly Asp
            20                  25                  30

agt ttg tcg caa aca cag ctg ctg gat cgc tta acc cat ggc tgc cgc 144
Ser Leu Ser Gln Thr Gln Leu Leu Asp Arg Leu Thr His Gly Cys Arg
        35                  40                  45

tac gat cgt ttg gaa cga ccc ata act tat tcg gaa act ggc cag cgg 192
Tyr Asp Arg Leu Glu Arg Pro Ile Thr Tyr Ser Glu Thr Gly Gln Arg
    50                  55                  60

cta ccg gtg gac gtc tat atg cgc gcc tac atc tac ttc atg cag aac 240
Leu Pro Val Asp Val Tyr Met Arg Ala Tyr Ile Tyr Phe Met Gln Asn
65                  70                  75                  80

ctg gaa gca cac gat cta cag ttc aag atc tat gcg ctc ctc caa atg    288
Leu Glu Ala His Asp Leu Gln Phe Lys Ile Tyr Ala Leu Leu Gln Met
                85                  90                  95

cga tac ctc gat ccg cgt ctc aac ttt cgc aac gta agt ccc aag cgc    336
Arg Tyr Leu Asp Pro Arg Leu Asn Phe Arg Asn Val Ser Pro Lys Arg
            100                 105                 110

agg cag ccg att ttg ggc gag gag cac ctg aga aac tcg ctc tgg atg    384
Arg Gln Pro Ile Leu Gly Glu Glu His Leu Arg Asn Ser Leu Trp Met
        115                 120                 125

ccc cac ata ttt cta gcc aat gag cgc gac tcc agt att ttg ggt ctc    432
Pro His Ile Phe Leu Ala Asn Glu Arg Asp Ser Ser Ile Leu Gly Leu
    130                 135                 140

acg gag aag gac atc ctc acc tcg ata tcg ccc gat ggc aca gtg atc    480
Thr Glu Lys Asp Ile Leu Thr Ser Ile Ser Pro Asp Gly Thr Val Ile
145                 150                 155                 160

gtt tcg aat cgc atc aag gcg aca ctc tat tgc tgg ctc aac ctt aag    528
Val Ser Asn Arg Ile Lys Ala Thr Leu Tyr Cys Trp Leu Asn Leu Lys
                165                 170                 175

aag ttc cca ttc gat gag cag cac tgc tcg acg gtg ctg gag agt tgg    576
Lys Phe Pro Phe Asp Glu Gln His Cys Ser Thr Val Leu Glu Ser Trp
            180                 185                 190

atg tac aat acg tcg gaa ctg gtc ctc cac tgg gag cag aag cgt ccg    624
Met Tyr Asn Thr Ser Glu Leu Val Leu His Trp Glu Gln Lys Arg Pro
        195                 200                 205

ata acc tac gat ggg gca ctg cat ctc acc gag ttt gtg ctg cag cgt    672
Ile Thr Tyr Asp Gly Ala Leu His Leu Thr Glu Phe Val Leu Gln Arg
    210                 215                 220

tcc tgg tca aac gag acg gta atc aat gcc gat ttg agt gac ctg cgt    720
Ser Trp Ser Asn Glu Thr Val Ile Asn Ala Asp Leu Ser Asp Leu Arg
225                 230                 235                 240

cat gga gcc ttt gcc ggc aac tac agc tcc ctc agc ttc acc gtt cac    768
His Gly Ala Phe Ala Gly Asn Tyr Ser Ser Leu Ser Phe Thr Val His
                245                 250                 255

ctg acg cga gtg gtt ggc ttt tac ctg atg gac tac ttt cta ccc tcc    816
Leu Thr Arg Val Val Gly Phe Tyr Leu Met Asp Tyr Phe Leu Pro Ser
            260                 265                 270

atg ctg att gtg gcc ata tcc tgg gta tcg ttc tgg ctg caa gca gat 864
Met Leu Ile Val Ala Ile Ser Trp Val Ser Phe Trp Leu Gln Ala Asp
        275                 280                 285

cag gcg ccg ccg cgc atc acg ctg ggc acc agc acc ctg ctg aca ttc 912
Gln Ala Pro Pro Arg Ile Thr Leu Gly Thr Ser Thr Leu Leu Thr Phe
    290                 295                 300

att acc ctg gcc tcg gca cag gga aag act ctg cca aag gtt agc tac 960
Ile Thr Leu Ala Ser Ala Gln Gly Lys Thr Leu Pro Lys Val Ser Tyr
305                 310                 315                 320

atc aag gtg tca gag gtg tgg ttc ctg ggc tgc acc atc ttc ata ttt 1008
Ile Lys Val Ser Glu Val Trp Phe Leu Gly Cys Thr Ile Phe Ile Phe
                325                 330                 335

ggc agc atg gtg gag ttc gcc ttt gtt aac acg att tgg cgt cgt aag 1056
Gly Ser Met Val Glu Phe Ala Phe Val Asn Thr Ile Trp Arg Arg Lys
            340                 345                 350

aag agt gtg ccc gtc aag aaa ctg aac agc aaa cac atc ctg aag tca 1104
Lys Ser Val Pro Val Lys Lys Leu Asn Ser Lys His Ile Leu Lys Ser
        355                 360                 365

act cta tcg ccg aat ctg ctc aga aga cgc agt cac gcc aga tcc aga 1152
Thr Leu Ser Pro Asn Leu Leu Arg Arg Arg Ser His Ala Arg Ser Arg
    370                 375                 380

tcc ttt tcg ggc acg gct ctg cat aag aca gcg gat acc ggc tcg ctg 1200
Ser Phe Ser Gly Thr Ala Leu His Lys Thr Ala Asp Thr Gly Ser Leu
385                 390                 395                 400

ggc ggt gcc ggt ttc aac aat tac cta acc gtc aat ttg ccc atc acc 1248
Gly Gly Ala Gly Phe Asn Asn Tyr Leu Thr Val Asn Leu Pro Ile Thr
                405                 410                 415

acg att aag gag ggc caa gtt ctg gat cag cag agg acc agc cgc gtc 1296
Thr Ile Lys Glu Gly Gln Val Leu Asp Gln Gln Arg Thr Ser Arg Val
            420                 425                 430

gtc cag aag atg gat gtt aac ttt gtg gag agt gcc ttt gga tcc atg 1344
Val Gln Lys Met Asp Val Asn Phe Val Glu Ser Ala Phe Gly Ser Met
        435                 440                 445

ggc tct gcc cag tcc act gga gcg atc agt gct gcc agc agc agc caa 1392
Gly Ser Ala Gln Ser Thr Gly Ala Ile Ser Ala Ala Ser Ser Ser Gln
    450                 455                 460

act ttg acc aac aat aac cga acg gag agc gcg ggg cac cag gag gag    1440
Thr Leu Thr Asn Asn Asn Arg Thr Glu Ser Ala Gly His Gln Glu Glu
465                 470                 475                 480

atc gat ctg agt ggc tgg acc acg cta acg ccc cag gaa ata gcc atg    1488
Ile Asp Leu Ser Gly Trp Thr Thr Leu Thr Pro Gln Glu Ile Ala Met
                485                 490                 495

tgg att gat agt cga gcc aga ttc gtt ttt ccg ctc tcc ttt ctc gtc    1536
Trp Ile Asp Ser Arg Ala Arg Phe Val Phe Pro Leu Ser Phe Leu Val
            500                 505                 510

ttt aat ctg ttt ttc tgg aca ttt gtc tac tgt att taa                1575
Phe Asn Leu Phe Phe Trp Thr Phe Val Tyr Cys Ile
        515                 520

<210> 2
<211> 524
<212> PRT
<213> Drosophila melanogaster

<400> 2
Met Glu Trp Ser Leu Ile Ile Ser Ile Leu Leu Asn Cys Leu Tyr Ala
1               5                   10                  15

Ser Ala Ser Ala Leu Asp Glu Asp Cys Pro Thr Leu Asp Asp Gly Asp
            20                  25                  30

Ser Leu Ser Gln Thr Gln Leu Leu Asp Arg Leu Thr His Gly Cys Arg
        35                  40                  45

Tyr Asp Arg Leu Glu Arg Pro Ile Thr Tyr Ser Glu Thr Gly Gln Arg
    50                  55                  60

Leu Pro Val Asp Val Tyr Met Arg Ala Tyr Ile Tyr Phe Met Gln Asn
65                  70                  75                  80

Leu Glu Ala His Asp Leu Gln Phe Lys Ile Tyr Ala Leu Leu Gln Met
                85                  90                  95

Arg Tyr Leu Asp Pro Arg Leu Asn Phe Arg Asn Val Ser Pro Lys Arg
            100                 105                 110

Arg Gln Pro Ile Leu Gly Glu Glu His Leu Arg Asn Ser Leu Trp Met
        115                 120                 125

Pro His Ile Phe Leu Ala Asn Glu Arg Asp Ser Ser Ile Leu Gly Leu

130                 135                 140

Thr Glu Lys Asp Ile Leu Thr Ser Ile Ser Pro Asp Gly Thr Val Ile
145                 150                 155                 160

Val Ser Asn Arg Ile Lys Ala Thr Leu Tyr Cys Trp Leu Asn Leu Lys
                165                 170                 175

Lys Phe Pro Phe Asp Glu Gln His Cys Ser Thr Val Leu Glu Ser Trp
            180                 185                 190

Met Tyr Asn Thr Ser Glu Leu Val Leu His Trp Glu Gln Lys Arg Pro
        195                 200                 205

Ile Thr Tyr Asp Gly Ala Leu His Leu Thr Glu Phe Val Leu Gln Arg
    210                 215                 220

Ser Trp Ser Asn Glu Thr Val Ile Asn Ala Asp Leu Ser Asp Leu Arg
225                 230                 235                 240

His Gly Ala Phe Ala Gly Asn Tyr Ser Ser Leu Ser Phe Thr Val His
                245                 250                 255

Leu Thr Arg Val Val Gly Phe Tyr Leu Met Asp Tyr Phe Leu Pro Ser
            260                 265                 270

Met Leu Ile Val Ala Ile Ser Trp Val Ser Phe Trp Leu Gln Ala Asp
        275                 280                 285

Gln Ala Pro Pro Arg Ile Thr Leu Gly Thr Ser Thr Leu Leu Thr Phe
    290                 295                 300

Ile Thr Leu Ala Ser Ala Gln Gly Lys Thr Leu Pro Lys Val Ser Tyr
305                 310                 315                 320

Ile Lys Val Ser Glu Val Trp Phe Leu Gly Cys Thr Ile Phe Ile Phe
                325                 330                 335

Gly Ser Met Val Glu Phe Ala Phe Val Asn Thr Ile Trp Arg Arg Lys
            340                 345                 350

Lys Ser Val Pro Val Lys Lys Leu Asn Ser Lys His Ile Leu Lys Ser
        355                 360                 365

Thr Leu Ser Pro Asn Leu Leu Arg Arg Arg Ser His Ala Arg Ser Arg
    370                 375                 380

Ser Phe Ser Gly Thr Ala Leu His Lys Thr Ala Asp Thr Gly Ser Leu

385                 390                 395                 400

Gly Gly Ala Gly Phe Asn Asn Tyr Leu Thr Val Asn Leu Pro Ile Thr
                405                 410                 415

Thr Ile Lys Glu Gly Gln Val Leu Asp Gln Gln Arg Thr Ser Arg Val
            420                 425                 430

Val Gln Lys Met Asp Val Asn Phe Val Glu Ser Ala Phe Gly Ser Met
        435                 440                 445

Gly Ser Ala Gln Ser Thr Gly Ala Ile Ser Ala Ala Ser Ser Ser Gln
    450                 455                 460

Thr Leu Thr Asn Asn Asn Arg Thr Glu Ser Ala Gly His Gln Glu Glu
465                 470                 475                 480

Ile Asp Leu Ser Gly Trp Thr Thr Leu Thr Pro Gln Glu Ile Ala Met
                485                 490                 495

Trp Ile Asp Ser Arg Ala Arg Phe Val Phe Pro Leu Ser Phe Leu Val
            500                 505                 510

Phe Asn Leu Phe Phe Trp Thr Phe Val Tyr Cys Ile
        515                 520

<210> 3
<211> 1269
<212> DNA
<213> Drosophila melanogaster

<220>
<221> CDS
<222> (1)..(1266)

<400> 3
atg caa agc cca act agc aaa ttg gta gaa ttc agg tgc ctg att gcg    48
Met Gln Ser Pro Thr Ser Lys Leu Val Glu Phe Arg Cys Leu Ile Ala
1               5                   10                  15

ttg gca ata tat ttg cac gcg ctg gag caa tcg atc cag cac tgc cat    96
Leu Ala Ile Tyr Leu His Ala Leu Glu Gln Ser Ile Gln His Cys His
            20                  25                  30

tgt gtt cat ggt tac aga aat aac acg gag agc gcc gag ctg gtc tcc    144
Cys Val His Gly Tyr Arg Asn Asn Thr Glu Ser Ala Glu Leu Val Ser

35                    40                    45

cac tac gag tcg agt ctt tcg ctc ccg gac att ttg ccc att ccc tca    192
His Tyr Glu Ser Ser Leu Ser Leu Pro Asp Ile Leu Pro Ile Pro Ser
    50                    55                    60

aag acg tac gac aag aac cgg gct ccc aag ctc ctc ggc cag ccc aca    240
Lys Thr Tyr Asp Lys Asn Arg Ala Pro Lys Leu Leu Gly Gln Pro Thr
65                    70                    75                    80

gta gtc tac ttc cat gtc acg gtc ctc tcc ctg gac tcc att aac gag    288
Val Val Tyr Phe His Val Thr Val Leu Ser Leu Asp Ser Ile Asn Glu
                85                    90                    95

gag tct atg acc tat gtg acg gac atc ttc ctt gca caa agc tgg cgt    336
Glu Ser Met Thr Tyr Val Thr Asp Ile Phe Leu Ala Gln Ser Trp Arg
            100                   105                   110

gat cct cgc ctg cgg ttg cct gag aac atg agt gag cag tat cgc ata    384
Asp Pro Arg Leu Arg Leu Pro Glu Asn Met Ser Glu Gln Tyr Arg Ile
        115                   120                   125

ttg gat gtc gac tgg ttg cac agc att tgg cgg ccc gat tgc ttc ttt    432
Leu Asp Val Asp Trp Leu His Ser Ile Trp Arg Pro Asp Cys Phe Phe
    130                   135                   140

aag aac gcc aaa aag gtc acc ttc cat gag atg agc att ccc aat cac    480
Lys Asn Ala Lys Lys Val Thr Phe His Glu Met Ser Ile Pro Asn His
145                   150                   155                   160

tat ctc tgg ctg tac cac gac aaa acg ctg ctc tat atg tcc aaa ctc    528
Tyr Leu Trp Leu Tyr His Asp Lys Thr Leu Leu Tyr Met Ser Lys Leu
                165                   170                   175

acg ttg gtc ctg tcg tgc gcc atg aag ttt gag tcc tat ccg cat gac    576
Thr Leu Val Leu Ser Cys Ala Met Lys Phe Glu Ser Tyr Pro His Asp
            180                   185                   190

acg caa atc tgc tcc atg atg atc gag agt tta tcc cat acg gtg gaa    624
Thr Gln Ile Cys Ser Met Met Ile Glu Ser Leu Ser His Thr Val Glu
        195                   200                   205

gat ttg gtt ttc att tgg aac atg acc gac cca ctt gtg gtt aac acg    672
Asp Leu Val Phe Ile Trp Asn Met Thr Asp Pro Leu Val Val Asn Thr
    210                   215                   220

gag att gag ttg ccg cag cta gac ata tca aat aac tac aca acc gac    720
Glu Ile Glu Leu Pro Gln Leu Asp Ile Ser Asn Asn Tyr Thr Thr Asp

225                 230                 235                 240

tgt act ata gag tac tca aca ggt aac ttc acc tgc ctg gcc att gtg     768
Cys Thr Ile Glu Tyr Ser Thr Gly Asn Phe Thr Cys Leu Ala Ile Val
                245                 250                 255

ttc aac ctg cgc cga cgc ctg ggt tac cat ttg ttc cac acc tac atc     816
Phe Asn Leu Arg Arg Arg Leu Gly Tyr His Leu Phe His Thr Tyr Ile
            260                 265                 270

ccc tcg gct ctg att gtg gtc atg tct tgg ata tcg ttt tgg ata aaa     864
Pro Ser Ala Leu Ile Val Val Met Ser Trp Ile Ser Phe Trp Ile Lys
        275                 280                 285

cca gaa gcg ata ccc gcc cgt gta act ctg gga gtg acc tca ctg cta     912
Pro Glu Ala Ile Pro Ala Arg Val Thr Leu Gly Val Thr Ser Leu Leu
    290                 295                 300

acc ctg gcc acc cag aat acc cag tcg caa caa tcg ctg ccg ccg gtt     960
Thr Leu Ala Thr Gln Asn Thr Gln Ser Gln Gln Ser Leu Pro Pro Val
305                 310                 315                 320

tcg tat gtc aag gct ata gac gtc tgg atg tcg tcc tgt tcg gtg ttt     1008
Ser Tyr Val Lys Ala Ile Asp Val Trp Met Ser Ser Cys Ser Val Phe
                325                 330                 335

gta ttc ctt tct ctg atg gaa ttt gca gtg gtc aac aat ttt atg gga     1056
Val Phe Leu Ser Leu Met Glu Phe Ala Val Val Asn Asn Phe Met Gly
            340                 345                 350

ccg gtg gcc aca aag gca atg aag ggg tat tcg gac gag aac atc agt     1104
Pro Val Ala Thr Lys Ala Met Lys Gly Tyr Ser Asp Glu Asn Ile Ser
        355                 360                 365

gat ctg gac gac cta aag cat cat cgg gaa tcg att att gag ccc cag     1152
Asp Leu Asp Asp Leu Lys His His Arg Glu Ser Ile Ile Glu Pro Gln
    370                 375                 380

ttc gac cct ttt tgc cat ggc cct gcc cca gcc ctt tat ata gac aaa     1200
Phe Asp Pro Phe Cys His Gly Pro Ala Pro Ala Leu Tyr Ile Asp Lys
385                 390                 395                 400

ttc tcg cgc ttt ttc ttc ccg ttt tcg ttc ttt ata ctc aat att gtc     1248
Phe Ser Arg Phe Phe Phe Pro Phe Ser Phe Phe Ile Leu Asn Ile Val
                405                 410                 415

tat tgg aca acg ttc cta tga                                         1269
Tyr Trp Thr Thr Phe Leu

            420

<210> 4
<211> 422
<212> PRT
<213> Drosophila melanogaster

<400> 4
Met Gln Ser Pro Thr Ser Lys Leu Val Glu Phe Arg Cys Leu Ile Ala
1               5                   10                  15

Leu Ala Ile Tyr Leu His Ala Leu Glu Gln Ser Ile Gln His Cys His
            20                  25                  30

Cys Val His Gly Tyr Arg Asn Asn Thr Glu Ser Ala Glu Leu Val Ser
        35                  40                  45

His Tyr Glu Ser Ser Leu Ser Leu Pro Asp Ile Leu Pro Ile Pro Ser
    50                  55                  60

Lys Thr Tyr Asp Lys Asn Arg Ala Pro Lys Leu Leu Gly Gln Pro Thr
65                  70                  75                  80

Val Val Tyr Phe His Val Thr Val Leu Ser Leu Asp Ser Ile Asn Glu
                85                  90                  95

Glu Ser Met Thr Tyr Val Thr Asp Ile Phe Leu Ala Gln Ser Trp Arg
            100                 105                 110

Asp Pro Arg Leu Arg Leu Pro Glu Asn Met Ser Glu Gln Tyr Arg Ile
        115                 120                 125

Leu Asp Val Asp Trp Leu His Ser Ile Trp Arg Pro Asp Cys Phe Phe
    130                 135                 140

Lys Asn Ala Lys Lys Val Thr Phe His Glu Met Ser Ile Pro Asn His
145                 150                 155                 160

Tyr Leu Trp Leu Tyr His Asp Lys Thr Leu Leu Tyr Met Ser Lys Leu
                165                 170                 175

Thr Leu Val Leu Ser Cys Ala Met Lys Phe Glu Ser Tyr Pro His Asp
            180                 185                 190

Thr Gln Ile Cys Ser Met Met Ile Glu Ser Leu Ser His Thr Val Glu
        195                 200                 205

Asp Leu Val Phe Ile Trp Asn Met Thr Asp Pro Leu Val Val Asn Thr
    210                 215                 220

Glu Ile Glu Leu Pro Gln Leu Asp Ile Ser Asn Asn Tyr Thr Thr Asp
225                 230                 235                 240

Cys Thr Ile Glu Tyr Ser Thr Gly Asn Phe Thr Cys Leu Ala Ile Val
                245                 250                 255

Phe Asn Leu Arg Arg Arg Leu Gly Tyr His Leu Phe His Thr Tyr Ile
            260                 265                 270

Pro Ser Ala Leu Ile Val Val Met Ser Trp Ile Ser Phe Trp Ile Lys
        275                 280                 285

Pro Glu Ala Ile Pro Ala Arg Val Thr Leu Gly Val Thr Ser Leu Leu
    290                 295                 300

Thr Leu Ala Thr Gln Asn Thr Gln Ser Gln Gln Ser Leu Pro Pro Val
305                 310                 315                 320

Ser Tyr Val Lys Ala Ile Asp Val Trp Met Ser Ser Cys Ser Val Phe
                325                 330                 335

Val Phe Leu Ser Leu Met Glu Phe Ala Val Val Asn Asn Phe Met Gly
            340                 345                 350

Pro Val Ala Thr Lys Ala Met Lys Gly Tyr Ser Asp Glu Asn Ile Ser
        355                 360                 365

Asp Leu Asp Asp Leu Lys His His Arg Glu Ser Ile Ile Glu Pro Gln
    370                 375                 380

Phe Asp Pro Phe Cys His Gly Pro Ala Pro Ala Leu Tyr Ile Asp Lys
385                 390                 395                 400

Phe Ser Arg Phe Phe Phe Pro Phe Ser Phe Phe Ile Leu Asn Ile Val
                405                 410                 415

Tyr Trp Thr Thr Phe Leu
            420

<210> 5
<211> 1192
<212> DNA
<213> Drosophila melanogaster

<220>
<221> CDS
<222> (1)..(1191)

<400> 5
atg caa agc cca act agc aaa ttg gta gaa ttc agg tgc ctg att gcg    48
Met Gln Ser Pro Thr Ser Lys Leu Val Glu Phe Arg Cys Leu Ile Ala
1               5                   10                  15

ttg gca ata tat ttg cac gcg ctg gag caa tcg atc cag cac tgc cat    96
Leu Ala Ile Tyr Leu His Ala Leu Glu Gln Ser Ile Gln His Cys His
            20                  25                  30

tgt gtt cat ggt tac aga aat aac acg gag agc gcc gag ctg gtc tcc    144
Cys Val His Gly Tyr Arg Asn Asn Thr Glu Ser Ala Glu Leu Val Ser
        35                  40                  45

cac tac gag tcg agt ctt tcg ctc ccg gac att ttg ccc att ccc tca    192
His Tyr Glu Ser Ser Leu Ser Leu Pro Asp Ile Leu Pro Ile Pro Ser
    50                  55                  60

aag acg tac gac aag aac cgg gct ccc aag ctc ctc ggc cag ccc aca    240
Lys Thr Tyr Asp Lys Asn Arg Ala Pro Lys Leu Leu Gly Gln Pro Thr
65                  70                  75                  80

gta gtc tac ttc cat gtc acg gtc ctc tcc ctg gac tcc att aac gag    288
Val Val Tyr Phe His Val Thr Val Leu Ser Leu Asp Ser Ile Asn Glu
                85                  90                  95

gag tct atg acc tat gtg acg gac atc ttc ctt gca caa agc tgg cgt    336
Glu Ser Met Thr Tyr Val Thr Asp Ile Phe Leu Ala Gln Ser Trp Arg
            100                 105                 110

gat cct cgc ctg cgg ttg cct gag aac atg agt gag cag tat cgc ata    384
Asp Pro Arg Leu Arg Leu Pro Glu Asn Met Ser Glu Gln Tyr Arg Ile
        115                 120                 125

ttg gat gtc gac tgg ttg cac agc att tgg cgg ccc gat tgc ttc ttt    432
Leu Asp Val Asp Trp Leu His Ser Ile Trp Arg Pro Asp Cys Phe Phe
    130                 135                 140

aag aac gcc aaa aag gtc acc ttc cat gag atg agc att ccc aat cac    480
Lys Asn Ala Lys Lys Val Thr Phe His Glu Met Ser Ile Pro Asn His
145                 150                 155                 160

tat ctc tgg ctg tac cac gac aaa acg ctg ctc tat atg tcc aaa ctc    528
Tyr Leu Trp Leu Tyr His Asp Lys Thr Leu Leu Tyr Met Ser Lys Leu

                165                 170                 175

acg ttg gtc ctg tcg tgc gcc atg aag ttt gag tcc tat ccg cat gac 576
Thr Leu Val Leu Ser Cys Ala Met Lys Phe Glu Ser Tyr Pro His Asp
            180                 185                 190

acg caa atc tgc tcc atg atg atc gag agt tta tcc cat acg gtg gaa 624
Thr Gln Ile Cys Ser Met Met Ile Glu Ser Leu Ser His Thr Val Glu
        195                 200                 205

gat ttg gtt ttc att tgg aac atg acc gac cca ctt gtg gtt aac acg 672
Asp Leu Val Phe Ile Trp Asn Met Thr Asp Pro Leu Val Val Asn Thr
    210                 215                 220

gag att gag ttg ccg cag cta gac ata tca aat aac tac aca acc gac 720
Glu Ile Glu Leu Pro Gln Leu Asp Ile Ser Asn Asn Tyr Thr Thr Asp
225                 230                 235                 240

tgt act ata gag tac tca aca ggt aac ttc acc tgc ctg gcc att gtg 768
Cys Thr Ile Glu Tyr Ser Thr Gly Asn Phe Thr Cys Leu Ala Ile Val
                245                 250                 255

ttc aac ctg cgc cga cgc ctg ggt tac cat ttg ttc cac acc tac atc 816
Phe Asn Leu Arg Arg Arg Leu Gly Tyr His Leu Phe His Thr Tyr Ile
            260                 265                 270

ccc tcg gct ctg att gtg gtc atg tct tgg ata tcg ttt tgg ata aaa 864
Pro Ser Ala Leu Ile Val Val Met Ser Trp Ile Ser Phe Trp Ile Lys
        275                 280                 285

cca gaa gcg ata ccc gcc cgt gta act ctg gga gtg acc tca ctg cta 912
Pro Glu Ala Ile Pro Ala Arg Val Thr Leu Gly Val Thr Ser Leu Leu
    290                 295                 300

acc ctg gcc acc cag aat acc cag tcg caa caa tcg ctg ccg ccg gtt 960
Thr Leu Ala Thr Gln Asn Thr Gln Ser Gln Gln Ser Leu Pro Pro Val
305                 310                 315                 320

tcg tat gtc aag gct ata gac gtc tgg atg tcg tcc tgt tcg gtg ttt 1008
Ser Tyr Val Lys Ala Ile Asp Val Trp Met Ser Ser Cys Ser Val Phe
                325                 330                 335

gta ttc ctt tct ctg atg gaa ttt gca gtg gtc aac aat ttt atg gga 1056
Val Phe Leu Ser Leu Met Glu Phe Ala Val Val Asn Asn Phe Met Gly
            340                 345                 350

ccg gtg gcc aca aag gca atg aag ggg tat tcg gac gag aac atc agt 1104
Pro Val Ala Thr Lys Ala Met Lys Gly Tyr Ser Asp Glu Asn Ile Ser

355                 360                 365

gat ctg gac gac cta aag cat cat cgg gaa tcg att att gag ccc cag    1152
Asp Leu Asp Asp Leu Lys His His Arg Glu Ser Ile Ile Glu Pro Gln
    370                 375                 380

tac gac act ttc tgc cat ggc cat gcc acg cca ttt ata t              1192
Tyr Asp Thr Phe Cys His Gly His Ala Thr Pro Phe Ile
385                 390                 395

<210> 6
<211> 397
<212> PRT
<213> Drosophila melanogaster

<400> 6
Met Gln Ser Pro Thr Ser Lys Leu Val Glu Phe Arg Cys Leu Ile Ala
  1               5                  10                  15

Leu Ala Ile Tyr Leu His Ala Leu Glu Gln Ser Ile Gln His Cys His
             20                  25                  30

Cys Val His Gly Tyr Arg Asn Asn Thr Glu Ser Ala Glu Leu Val Ser
         35                  40                  45

His Tyr Glu Ser Ser Leu Ser Leu Pro Asp Ile Leu Pro Ile Pro Ser
     50                  55                  60

Lys Thr Tyr Asp Lys Asn Arg Ala Pro Lys Leu Leu Gly Gln Pro Thr
 65                  70                  75                  80

Val Val Tyr Phe His Val Thr Val Leu Ser Leu Asp Ser Ile Asn Glu
                 85                  90                  95

Glu Ser Met Thr Tyr Val Thr Asp Ile Phe Leu Ala Gln Ser Trp Arg
            100                 105                 110

Asp Pro Arg Leu Arg Leu Pro Glu Asn Met Ser Glu Gln Tyr Arg Ile
        115                 120                 125

Leu Asp Val Asp Trp Leu His Ser Ile Trp Arg Pro Asp Cys Phe Phe
    130                 135                 140

Lys Asn Ala Lys Lys Val Thr Phe His Glu Met Ser Ile Pro Asn His
145                 150                 155                 160

Tyr Leu Trp Leu Tyr His Asp Lys Thr Leu Leu Tyr Met Ser Lys Leu

165                 170                 175

Thr Leu Val Leu Ser Cys Ala Met Lys Phe Glu Ser Tyr Pro His Asp
            180                 185                 190

Thr Gln Ile Cys Ser Met Met Ile Glu Ser Leu Ser His Thr Val Glu
        195                 200                 205

Asp Leu Val Phe Ile Trp Asn Met Thr Asp Pro Leu Val Val Asn Thr
    210                 215                 220

Glu Ile Glu Leu Pro Gln Leu Asp Ile Ser Asn Asn Tyr Thr Thr Asp
225                 230                 235                 240

Cys Thr Ile Glu Tyr Ser Thr Gly Asn Phe Thr Cys Leu Ala Ile Val
                245                 250                 255

Phe Asn Leu Arg Arg Arg Leu Gly Tyr His Leu Phe His Thr Tyr Ile
            260                 265                 270

Pro Ser Ala Leu Ile Val Val Met Ser Trp Ile Ser Phe Trp Ile Lys
        275                 280                 285

Pro Glu Ala Ile Pro Ala Arg Val Thr Leu Gly Val Thr Ser Leu Leu
    290                 295                 300

Thr Leu Ala Thr Gln Asn Thr Gln Ser Gln Gln Ser Leu Pro Pro Val
305                 310                 315                 320

Ser Tyr Val Lys Ala Ile Asp Val Trp Met Ser Ser Cys Ser Val Phe
                325                 330                 335

Val Phe Leu Ser Leu Met Glu Phe Ala Val Val Asn Asn Phe Met Gly
            340                 345                 350

Pro Val Ala Thr Lys Ala Met Lys Gly Tyr Ser Asp Glu Asn Ile Ser
        355                 360                 365

Asp Leu Asp Asp Leu Lys His His Arg Glu Ser Ile Ile Glu Pro Gln
    370                 375                 380

Tyr Asp Thr Phe Cys His Gly His Ala Thr Pro Phe Ile
385                 390                 395

<210> 7
<211> 1272

<212> DNA
<213> Drosophila melanogaster

<220>
<221> CDS
<222> (1)..(1269)

<400> 7
atg gag tcc gag tgc gtt tgc cca gtt tca tca gat tta cgc cac agg    48
Met Glu Ser Glu Cys Val Cys Pro Val Ser Ser Asp Leu Arg His Arg
1               5                   10                  15

gct gtt ctg ttc cac ata tgc aga aat aac acg gag agc gcc gag ctg    96
Ala Val Leu Phe His Ile Cys Arg Asn Asn Thr Glu Ser Ala Glu Leu
            20                  25                  30

gtc tcc cac tac gag tcg agt ctt tcg ctc ccg gac att ttg ccc att    144
Val Ser His Tyr Glu Ser Ser Leu Ser Leu Pro Asp Ile Leu Pro Ile
        35                  40                  45

ccc tca aag acg tac gac aag aac cgg gct ccc aag ctc ctc ggc cag    192
Pro Ser Lys Thr Tyr Asp Lys Asn Arg Ala Pro Lys Leu Leu Gly Gln
    50                  55                  60

ccc aca gta gtc tac ttc cat gtc acg gtc ctc tcc ctg gac tcc att    240
Pro Thr Val Val Tyr Phe His Val Thr Val Leu Ser Leu Asp Ser Ile
65                  70                  75                  80

aac gag gag tct atg acc tat gtg acg gac atc ttc ctt gca caa agc    288
Asn Glu Glu Ser Met Thr Tyr Val Thr Asp Ile Phe Leu Ala Gln Ser
                85                  90                  95

tgg cgt gat cct cgc ctg cgg ttg cct gag aac atg agt gag cag tat    336
Trp Arg Asp Pro Arg Leu Arg Leu Pro Glu Asn Met Ser Glu Gln Tyr
            100                 105                 110

cgc ata ttg gat gtc gac tgg ttg cac agc att tgg cgg ccc gat tgc    384
Arg Ile Leu Asp Val Asp Trp Leu His Ser Ile Trp Arg Pro Asp Cys
        115                 120                 125

ttc ttt aag aac gcc aaa aag gtc acc ttc cat gag atg agc att ccc    432
Phe Phe Lys Asn Ala Lys Lys Val Thr Phe His Glu Met Ser Ile Pro
    130                 135                 140

aat cac tat ctc tgg ctg tac cac gac aaa acg ctg ctc tat atg tcc    480
Asn His Tyr Leu Trp Leu Tyr His Asp Lys Thr Leu Leu Tyr Met Ser
145                 150                 155                 160

aaa ctc acg ttg gtc ctg tcg tgc gcc atg aag ttt gag tcc tat ccg    528
Lys Leu Thr Leu Val Leu Ser Cys Ala Met Lys Phe Glu Ser Tyr Pro
                165                 170                 175

cat gac acg caa atc tgc tcc atg atg atc gag agt tta tcc cat acg    576
His Asp Thr Gln Ile Cys Ser Met Met Ile Glu Ser Leu Ser His Thr
            180                 185                 190

gtg gaa gat ttg gtt ttc att tgg aac atg acc gac cca ctt gtg gtt    624
Val Glu Asp Leu Val Phe Ile Trp Asn Met Thr Asp Pro Leu Val Val
        195                 200                 205

aac acg gag att gag ttg ccg cag cta gac ata tca aat aac tac aca    672
Asn Thr Glu Ile Glu Leu Pro Gln Leu Asp Ile Ser Asn Asn Tyr Thr
    210                 215                 220

acc gac tgt act ata gag tac tca aca ggt aac ttc acc tgc ctg gcc    720
Thr Asp Cys Thr Ile Glu Tyr Ser Thr Gly Asn Phe Thr Cys Leu Ala
225                 230                 235                 240

att gtg ttc aac ctg cgc cga cgc ctg ggt tac cat ttg ttc cac acc    768
Ile Val Phe Asn Leu Arg Arg Arg Leu Gly Tyr His Leu Phe His Thr
                245                 250                 255

tac atc ccc tcg gct ctg att gtg gtc atg tct tgg ata tcg ttt tgg    816
Tyr Ile Pro Ser Ala Leu Ile Val Val Met Ser Trp Ile Ser Phe Trp
            260                 265                 270

ata aaa cca gaa gcg ata ccc gcc cgt gta act ctg gga gtg acc tca    864
Ile Lys Pro Glu Ala Ile Pro Ala Arg Val Thr Leu Gly Val Thr Ser
        275                 280                 285

ctg cta acc ctg gcc acc cag aat acc cag tcg caa caa tcg ctg ccg    912
Leu Leu Thr Leu Ala Thr Gln Asn Thr Gln Ser Gln Gln Ser Leu Pro
    290                 295                 300

ccg gtt tcg tat gtc aag gct ata gac gtc tgg atg tcg tcc tgt tcg    960
Pro Val Ser Tyr Val Lys Ala Ile Asp Val Trp Met Ser Ser Cys Ser
305                 310                 315                 320

gtg ttt gta ttc ctt tct ctg atg gaa ttt gca gtg gtc aac aat ttt    1008
Val Phe Val Phe Leu Ser Leu Met Glu Phe Ala Val Val Asn Asn Phe
                325                 330                 335

atg gga ccg gtg gcc aca aag gca atg aag ggg tat tcg gac gag aac    1056
Met Gly Pro Val Ala Thr Lys Ala Met Lys Gly Tyr Ser Asp Glu Asn
            340                 345                 350

atc agt gat ctg gac gac cta aag gtt gtg cgg cag tta cag att gga    1104
Ile Ser Asp Leu Asp Asp Leu Lys Val Val Arg Gln Leu Gln Ile Gly
        355                 360                 365

ctt cag cca gtt ctg aaa aag aag tta agc aat tac ttc aaa gtt cct    1152
Leu Gln Pro Val Leu Lys Lys Lys Leu Ser Asn Tyr Phe Lys Val Pro
    370                 375                 380

aaa gcc ata ata aat gta ttt acc aat gat gcc ctt cgg agc ggt atc    1200
Lys Ala Ile Ile Asn Val Phe Thr Asn Asp Ala Leu Arg Ser Gly Ile
385                 390                 395                 400

ttc gga ggt gtc ata gta acg aag ttg atg ctt aat aga gtc caa cga    1248
Phe Gly Gly Val Ile Val Thr Lys Leu Met Leu Asn Arg Val Gln Arg
                405                 410                 415

acc atc gct gtt tcc agc cct tga                                    1272
Thr Ile Ala Val Ser Ser Pro
            420

<210> 8
<211> 423
<212> PRT
<213> Drosophila melanogaster

<400> 8
Met Glu Ser Glu Cys Val Cys Pro Val Ser Ser Asp Leu Arg His Arg
 1               5                  10                  15

Ala Val Leu Phe His Ile Cys Arg Asn Asn Thr Glu Ser Ala Glu Leu
            20                  25                  30

Val Ser His Tyr Glu Ser Ser Leu Ser Leu Pro Asp Ile Leu Pro Ile
        35                  40                  45

Pro Ser Lys Thr Tyr Asp Lys Asn Arg Ala Pro Lys Leu Leu Gly Gln
    50                  55                  60

Pro Thr Val Val Tyr Phe His Val Thr Val Leu Ser Leu Asp Ser Ile
65                  70                  75                  80

Asn Glu Glu Ser Met Thr Tyr Val Thr Asp Ile Phe Leu Ala Gln Ser
                85                  90                  95

Trp Arg Asp Pro Arg Leu Arg Leu Pro Glu Asn Met Ser Glu Gln Tyr
            100                 105                 110

Arg Ile Leu Asp Val Asp Trp Leu His Ser Ile Trp Arg Pro Asp Cys
        115                 120                 125

Phe Phe Lys Asn Ala Lys Lys Val Thr Phe His Glu Met Ser Ile Pro
    130                 135                 140

Asn His Tyr Leu Trp Leu Tyr His Asp Lys Thr Leu Leu Tyr Met Ser
145                 150                 155                 160

Lys Leu Thr Leu Val Leu Ser Cys Ala Met Lys Phe Glu Ser Tyr Pro
                165                 170                 175

His Asp Thr Gln Ile Cys Ser Met Met Ile Glu Ser Leu Ser His Thr
            180                 185                 190

Val Glu Asp Leu Val Phe Ile Trp Asn Met Thr Asp Pro Leu Val Val
        195                 200                 205

Asn Thr Glu Ile Glu Leu Pro Gln Leu Asp Ile Ser Asn Asn Tyr Thr
    210                 215                 220

Thr Asp Cys Thr Ile Glu Tyr Ser Thr Gly Asn Phe Thr Cys Leu Ala
225                 230                 235                 240

Ile Val Phe Asn Leu Arg Arg Arg Leu Gly Tyr His Leu Phe His Thr
                245                 250                 255

Tyr Ile Pro Ser Ala Leu Ile Val Val Met Ser Trp Ile Ser Phe Trp
            260                 265                 270

Ile Lys Pro Glu Ala Ile Pro Ala Arg Val Thr Leu Gly Val Thr Ser
        275                 280                 285

Leu Leu Thr Leu Ala Thr Gln Asn Thr Gln Ser Gln Gln Ser Leu Pro
    290                 295                 300

Pro Val Ser Tyr Val Lys Ala Ile Asp Val Trp Met Ser Ser Cys Ser
305                 310                 315                 320

Val Phe Val Phe Leu Ser Leu Met Glu Phe Ala Val Val Asn Asn Phe
                325                 330                 335

Met Gly Pro Val Ala Thr Lys Ala Met Lys Gly Tyr Ser Asp Glu Asn
            340                 345                 350

Ile Ser Asp Leu Asp Asp Leu Lys Val Val Arg Gln Leu Gln Ile Gly
        355                 360                 365

Leu Gln Pro Val Leu Lys Lys Lys Leu Ser Asn Tyr Phe Lys Val Pro
    370                 375                 380

Lys Ala Ile Ile Asn Val Phe Thr Asn Asp Ala Leu Arg Ser Gly Ile
385                 390                 395                 400

Phe Gly Gly Val Ile Val Thr Lys Leu Met Leu Asn Arg Val Gln Arg
                405                 410                 415

Thr Ile Ala Val Ser Ser Pro
            420

<210> 9
<211> 1608
<212> DNA
<213> Drosophila melanogaster

<220>
<221> CDS
<222> (1)..(1605)

<400> 9
atg gtg ttc caa ata ata atc ctg gtg atc tgc acc atc tgc atg aag    48
Met Val Phe Gln Ile Ile Ile Leu Val Ile Cys Thr Ile Cys Met Lys
  1               5                  10                  15

cac tac gcc aaa ggg gag ttt caa caa agt ctg gcc ata acc gac atc    96
His Tyr Ala Lys Gly Glu Phe Gln Gln Ser Leu Ala Ile Thr Asp Ile
             20                  25                  30

ctg ccc gag gac atc aag cgt tac gac aag atg aga ccg ccg aag aaa    144
Leu Pro Glu Asp Ile Lys Arg Tyr Asp Lys Met Arg Pro Pro Lys Lys
         35                  40                  45

gag ggt cag ccg acg ata gtc tac ttc cat gtg acg gtg atg ggt ctg    192
Glu Gly Gln Pro Thr Ile Val Tyr Phe His Val Thr Val Met Gly Leu
     50                  55                  60

gac tcc att gat gag aac tcg atg act tat gtg gcg gat gtg ttc ttt    240
Asp Ser Ile Asp Glu Asn Ser Met Thr Tyr Val Ala Asp Val Phe Phe
 65                  70                  75                  80

gca cag acg tgg aag gat cat cgc ctg cga ttg ccg gag aat atg aca    288
Ala Gln Thr Trp Lys Asp His Arg Leu Arg Leu Pro Glu Asn Met Thr
                 85                  90                  95

cag gaa tac cgc ctg ctc gag gtg gac tgg cta aaa aat atg tgg cgg 336
Gln Glu Tyr Arg Leu Leu Glu Val Asp Trp Leu Lys Asn Met Trp Arg
            100                 105                 110

ccg gat tcg ttt ttc aaa aac gcc aaa tcg gtg acc ttt cag acc atg 384
Pro Asp Ser Phe Phe Lys Asn Ala Lys Ser Val Thr Phe Gln Thr Met
        115                 120                 125

aca ata ccc aat cac tat atg tgg ctg tac aag gat aag acc att ctg 432
Thr Ile Pro Asn His Tyr Met Trp Leu Tyr Lys Asp Lys Thr Ile Leu
    130                 135                 140

tac atg gtc aaa cta aca ctg aag ctg tcc tgc atc atg aac ttc gcc 480
Tyr Met Val Lys Leu Thr Leu Lys Leu Ser Cys Ile Met Asn Phe Ala
145                 150                 155                 160

att tat cct cat gat aca cag gag tgc aag ctg caa atg gaa agc ctg 528
Ile Tyr Pro His Asp Thr Gln Glu Cys Lys Leu Gln Met Glu Ser Leu
                165                 170                 175

tcc cac acc acg gat gac ttg ata ttc cag tgg gat cca aca acg ccc 576
Ser His Thr Thr Asp Asp Leu Ile Phe Gln Trp Asp Pro Thr Thr Pro
            180                 185                 190

ctt gtg gtt gat gaa aac atc gaa ctg ccg cag gtg gcc ctc atc cga 624
Leu Val Val Asp Glu Asn Ile Glu Leu Pro Gln Val Ala Leu Ile Arg
        195                 200                 205

aat gaa acg gcg gac tgt acc caa gtt tat tcc act ggc aac ttc aca 672
Asn Glu Thr Ala Asp Cys Thr Gln Val Tyr Ser Thr Gly Asn Phe Thr
    210                 215                 220

tgc ctg gag gtg gtg ttc acc ctt aag cgt cgt ttg gtt tac tac gtt 720
Cys Leu Glu Val Val Phe Thr Leu Lys Arg Arg Leu Val Tyr Tyr Val
225                 230                 235                 240

ttc aac acc tac att ccc acc tgc atg ata gtg atc atg tca tgg gta 768
Phe Asn Thr Tyr Ile Pro Thr Cys Met Ile Val Ile Met Ser Trp Val
                245                 250                 255

tcc ttc tgg atc aaa ccg gag gcg gca cca gcc cgt gtg act ctg ggt 816
Ser Phe Trp Ile Lys Pro Glu Ala Ala Pro Ala Arg Val Thr Leu Gly
            260                 265                 270

gtc acc tcc ttg cta acg ctt tcc acg caa cac gcc aaa tcg cag tcg 864
Val Thr Ser Leu Leu Thr Leu Ser Thr Gln His Ala Lys Ser Gln Ser
        275                 280                 285

tct ttg cca cct gtt tcc tat ctc aag gca gtg gac gcc ttt atg tcc 912
Ser Leu Pro Pro Val Ser Tyr Leu Lys Ala Val Asp Ala Phe Met Ser
    290                 295                 300

gtt tgc acg gtg ttc gtg ttt atg gcc ctc atg gag tat tgt cta ata 960
Val Cys Thr Val Phe Val Phe Met Ala Leu Met Glu Tyr Cys Leu Ile
305                 310                 315                 320

aac atc gtc ctg agc gac acg ccc att ccc aag ccg atg gcc tat cca 1008
Asn Ile Val Leu Ser Asp Thr Pro Ile Pro Lys Pro Met Ala Tyr Pro
                325                 330                 335

ccc aaa cct gtg gcg gga gat ggg ccc aag aaa gag ggc gag ggt gct 1056
Pro Lys Pro Val Ala Gly Asp Gly Pro Lys Lys Glu Gly Glu Gly Ala
            340                 345                 350

cct cct ggg ggc agc aac tcg acg gcc agc aag caa caa gcc acc atg 1104
Pro Pro Gly Gly Ser Asn Ser Thr Ala Ser Lys Gln Gln Ala Thr Met
        355                 360                 365

ttg cca ctg gcc gat gaa aag atc gag aaa att gag aag atc ttt gac 1152
Leu Pro Leu Ala Asp Glu Lys Ile Glu Lys Ile Glu Lys Ile Phe Asp
    370                 375                 380

gag atg acc aag aat aga agg att gta acc acc aca cgc cgc gtg gtg 1200
Glu Met Thr Lys Asn Arg Arg Ile Val Thr Thr Thr Arg Arg Val Val
385                 390                 395                 400

cgt ccg cca ttg gac gcc gat ggt ccg tgg att ccg cga cag gag tcg 1248
Arg Pro Pro Leu Asp Ala Asp Gly Pro Trp Ile Pro Arg Gln Glu Ser
                405                 410                 415

cgg ata ata ctg acc ccg act atc gct ccg ccg cca ccg ccc cct cag 1296
Arg Ile Ile Leu Thr Pro Thr Ile Ala Pro Pro Pro Pro Pro Pro Gln
            420                 425                 430

cca gcg gca ccg gaa ccg gaa ctg ccc aag ccg aaa ctc aca ccc gcc 1344
Pro Ala Ala Pro Glu Pro Glu Leu Pro Lys Pro Lys Leu Thr Pro Ala
        435                 440                 445

cag gag cgg ctc aag cgg gct ata tat ata gat cgg tcc tcg cgc gtc 1392
Gln Glu Arg Leu Lys Arg Ala Ile Tyr Ile Asp Arg Ser Ser Arg Val
    450                 455                 460

ctt ttc ccc gcc ctc ttc gcc agt ctg aat ggc atc tac tgg tgt caa 1440
Leu Phe Pro Ala Leu Phe Ala Ser Leu Asn Gly Ile Tyr Trp Cys Gln
465                 470                 475                 480

ctt tgg atg gaa caa tgg aac ttg ctc gat ttt cta ccg cct gca gtc    1488
Leu Trp Met Glu Gln Trp Asn Leu Leu Asp Phe Leu Pro Pro Ala Val
                485                 490                 495

ttc aaa cag ccg cag ggt gaa gag aaa cag act tat gaa tac cca att    1536
Phe Lys Gln Pro Gln Gly Glu Glu Lys Gln Thr Tyr Glu Tyr Pro Ile
            500                 505                 510

aat tat gtt ggt cac acg ctc cac act ttt gcc ttt tca cgg tcg tta    1584
Asn Tyr Val Gly His Thr Leu His Thr Phe Ala Phe Ser Arg Ser Leu
        515                 520                 525

gtg acg ctc gag caa aaa cta tga                                    1608
Val Thr Leu Glu Gln Lys Leu
    530                 535

<210> 10
<211> 535
<212> PRT
<213> Drosophila melanogaster

<400> 10
Met Val Phe Gln Ile Ile Ile Leu Val Ile Cys Thr Ile Cys Met Lys
1               5                   10                  15

His Tyr Ala Lys Gly Glu Phe Gln Gln Ser Leu Ala Ile Thr Asp Ile
            20                  25                  30

Leu Pro Glu Asp Ile Lys Arg Tyr Asp Lys Met Arg Pro Pro Lys Lys
        35                  40                  45

Glu Gly Gln Pro Thr Ile Val Tyr Phe His Val Thr Val Met Gly Leu
    50                  55                  60

Asp Ser Ile Asp Glu Asn Ser Met Thr Tyr Val Ala Asp Val Phe Phe
65                  70                  75                  80

Ala Gln Thr Trp Lys Asp His Arg Leu Arg Leu Pro Glu Asn Met Thr
                85                  90                  95

Gln Glu Tyr Arg Leu Leu Glu Val Asp Trp Leu Lys Asn Met Trp Arg
            100                 105                 110

Pro Asp Ser Phe Phe Lys Asn Ala Lys Ser Val Thr Phe Gln Thr Met
        115                 120                 125

Thr Ile Pro Asn His Tyr Met Trp Leu Tyr Lys Asp Lys Thr Ile Leu

130                 135                 140

Tyr Met Val Lys Leu Thr Leu Lys Leu Ser Cys Ile Met Asn Phe Ala
145                 150                 155                 160

Ile Tyr Pro His Asp Thr Gln Glu Cys Lys Leu Gln Met Glu Ser Leu
                165                 170                 175

Ser His Thr Thr Asp Asp Leu Ile Phe Gln Trp Asp Pro Thr Thr Pro
            180                 185                 190

Leu Val Val Asp Glu Asn Ile Glu Leu Pro Gln Val Ala Leu Ile Arg
        195                 200                 205

Asn Glu Thr Ala Asp Cys Thr Gln Val Tyr Ser Thr Gly Asn Phe Thr
    210                 215                 220

Cys Leu Glu Val Val Phe Thr Leu Lys Arg Arg Leu Val Tyr Tyr Val
225                 230                 235                 240

Phe Asn Thr Tyr Ile Pro Thr Cys Met Ile Val Ile Met Ser Trp Val
                245                 250                 255

Ser Phe Trp Ile Lys Pro Glu Ala Ala Pro Ala Arg Val Thr Leu Gly
            260                 265                 270

Val Thr Ser Leu Leu Thr Leu Ser Thr Gln His Ala Lys Ser Gln Ser
        275                 280                 285

Ser Leu Pro Pro Val Ser Tyr Leu Lys Ala Val Asp Ala Phe Met Ser
    290                 295                 300

Val Cys Thr Val Phe Val Phe Met Ala Leu Met Glu Tyr Cys Leu Ile
305                 310                 315                 320

Asn Ile Val Leu Ser Asp Thr Pro Ile Pro Lys Pro Met Ala Tyr Pro
                325                 330                 335

Pro Lys Pro Val Ala Gly Asp Gly Pro Lys Lys Glu Gly Glu Gly Ala
            340                 345                 350

Pro Pro Gly Gly Ser Asn Ser Thr Ala Ser Lys Gln Gln Ala Thr Met
        355                 360                 365

Leu Pro Leu Ala Asp Glu Lys Ile Glu Lys Ile Glu Lys Ile Phe Asp
    370                 375                 380

Glu Met Thr Lys Asn Arg Arg Ile Val Thr Thr Thr Arg Arg Val Val

385                 390                 395                 400

Arg Pro Pro Leu Asp Ala Asp Gly Pro Trp Ile Pro Arg Gln Glu Ser
                405                 410                 415

Arg Ile Ile Leu Thr Pro Thr Ile Ala Pro Pro Pro Pro Pro Pro Gln
            420                 425                 430

Pro Ala Ala Pro Glu Pro Glu Leu Pro Lys Pro Lys Leu Thr Pro Ala
        435                 440                 445

Gln Glu Arg Leu Lys Arg Ala Ile Tyr Ile Asp Arg Ser Ser Arg Val
    450                 455                 460

Leu Phe Pro Ala Leu Phe Ala Ser Leu Asn Gly Ile Tyr Trp Cys Gln
465                 470                 475                 480

Leu Trp Met Glu Gln Trp Asn Leu Leu Asp Phe Leu Pro Pro Ala Val
                485                 490                 495

Phe Lys Gln Pro Gln Gly Glu Glu Lys Gln Thr Tyr Glu Tyr Pro Ile
            500                 505                 510

Asn Tyr Val Gly His Thr Leu His Thr Phe Ala Phe Ser Arg Ser Leu
        515                 520                 525

Val Thr Leu Glu Gln Lys Leu
    530                 535

<210> 11
<211> 1458
<212> DNA
<213> Drosophila melanogaster

<220>
<221> CDS
<222> (1)..(1455)

<400> 11
atg gtg ttc caa ata ata atc ctg gtg atc tgc acc atc tgc atg aag    48
Met Val Phe Gln Ile Ile Ile Leu Val Ile Cys Thr Ile Cys Met Lys
  1               5                  10                  15

cac tac gcc aaa ggg gag ttt caa caa agt ctg gcc ata acc gac atc    96
His Tyr Ala Lys Gly Glu Phe Gln Gln Ser Leu Ala Ile Thr Asp Ile
             20                  25                  30

ctg ccc gag gac atc aag cgt tac gac aag atg aga ccg ccg aag aaa 144
Leu Pro Glu Asp Ile Lys Arg Tyr Asp Lys Met Arg Pro Pro Lys Lys
        35                  40                  45

gag ggt cag ccg acg ata gtc tac ttc cat gtg acg gtg atg ggt ctg 192
Glu Gly Gln Pro Thr Ile Val Tyr Phe His Val Thr Val Met Gly Leu
    50                  55                  60

gac tcc att gat gag aac tcg atg act tat gtg gcg gat gtg ttc ttt 240
Asp Ser Ile Asp Glu Asn Ser Met Thr Tyr Val Ala Asp Val Phe Phe
65                  70                  75                  80

gca cag acg tgg aag gat cat cgc ctg cga ttg ccg gag aat atg aca 288
Ala Gln Thr Trp Lys Asp His Arg Leu Arg Leu Pro Glu Asn Met Thr
                85                  90                  95

cag gaa tac cgc ctg ctc gag gtg gac tgg cta aaa aat atg tgg cgg 336
Gln Glu Tyr Arg Leu Leu Glu Val Asp Trp Leu Lys Asn Met Trp Arg
            100                 105                 110

ccg gat tcg ttt ttc aaa aac gcc aaa tcg gtg acc ttt cag acc atg 384
Pro Asp Ser Phe Phe Lys Asn Ala Lys Ser Val Thr Phe Gln Thr Met
        115                 120                 125

aca ata ccc aat cac tat atg tgg ctg tac aag gat aag acc att ctg 432
Thr Ile Pro Asn His Tyr Met Trp Leu Tyr Lys Asp Lys Thr Ile Leu
    130                 135                 140

tac atg gtc aaa cta aca ctg aag ctg tcc tgc atc atg aac ttc gcc 480
Tyr Met Val Lys Leu Thr Leu Lys Leu Ser Cys Ile Met Asn Phe Ala
145                 150                 155                 160

att tat cct cat gat aca cag gag tgc aag ctg caa atg gaa agc ctg 528
Ile Tyr Pro His Asp Thr Gln Glu Cys Lys Leu Gln Met Glu Ser Leu
                165                 170                 175

tcc cac acc acg gat gac ttg ata ttc cag tgg gat cca aca acg ccc 576
Ser His Thr Thr Asp Asp Leu Ile Phe Gln Trp Asp Pro Thr Thr Pro
            180                 185                 190

ctt gtg gtt gat gaa aac atc gaa ctg ccg cag gtg gcc ctc atc cga 624
Leu Val Val Asp Glu Asn Ile Glu Leu Pro Gln Val Ala Leu Ile Arg
        195                 200                 205

aat gaa acg gcg gac tgt acc caa gtt tat tcc act ggc aac ttc aca 672
Asn Glu Thr Ala Asp Cys Thr Gln Val Tyr Ser Thr Gly Asn Phe Thr
    210                 215                 220

tgc ctg gag gtg gtg ttc acc ctt aag cgt cgt ttg gtt tac tac gtt    720
Cys Leu Glu Val Val Phe Thr Leu Lys Arg Arg Leu Val Tyr Tyr Val
225                 230                 235                 240

ttc aac acc tac att ccc acc tgc atg ata gtg atc atg tca tgg gta    768
Phe Asn Thr Tyr Ile Pro Thr Cys Met Ile Val Ile Met Ser Trp Val
                245                 250                 255

tcc ttc tgg atc aaa ccg gag gcg gca cca gcc cgt gtg act ctg ggt    816
Ser Phe Trp Ile Lys Pro Glu Ala Ala Pro Ala Arg Val Thr Leu Gly
            260                 265                 270

gtc acc tcc ttg cta acg ctt tcc acg caa cac gcc aaa tcg cag tcg    864
Val Thr Ser Leu Leu Thr Leu Ser Thr Gln His Ala Lys Ser Gln Ser
        275                 280                 285

tct ttg cca cct gtt tcc tat ctc aag gca gtg gac gcc ttt atg tcc    912
Ser Leu Pro Pro Val Ser Tyr Leu Lys Ala Val Asp Ala Phe Met Ser
    290                 295                 300

gtt tgc acg gtg ttc gtg ttt atg gcc ctc atg gag tat tgt cta ata    960
Val Cys Thr Val Phe Val Phe Met Ala Leu Met Glu Tyr Cys Leu Ile
305                 310                 315                 320

aac atc gtc ctg agc gac acg ccc att ccc aag ccg atg gcc tat cca    1008
Asn Ile Val Leu Ser Asp Thr Pro Ile Pro Lys Pro Met Ala Tyr Pro
                325                 330                 335

ccc aaa cct gtg gcg gga gat ggg ccc aag aaa gag ggc gag ggt gct    1056
Pro Lys Pro Val Ala Gly Asp Gly Pro Lys Lys Glu Gly Glu Gly Ala
            340                 345                 350

cct cct ggg ggc agc aac tcg acg gcc agc aag caa caa gcc acc atg    1104
Pro Pro Gly Gly Ser Asn Ser Thr Ala Ser Lys Gln Gln Ala Thr Met
        355                 360                 365

ttg cca ctg gcc gat gaa aag atc gag aaa att gag aag atc ttt gac    1152
Leu Pro Leu Ala Asp Glu Lys Ile Glu Lys Ile Glu Lys Ile Phe Asp
    370                 375                 380

gag atg acc aag aat aga agg att gta acc acc aca cgc cgc gtg gtg    1200
Glu Met Thr Lys Asn Arg Arg Ile Val Thr Thr Thr Arg Arg Val Val
385                 390                 395                 400

cgt ccg cca ttg gac gcc gat ggt ccg tgg att ccg cga cag gag tcg    1248
Arg Pro Pro Leu Asp Ala Asp Gly Pro Trp Ile Pro Arg Gln Glu Ser
                405                 410                 415

cgg ata ata ctg acc ccg act atc gct ccg ccg cca ccg ccc cct cag    1296
Arg Ile Ile Leu Thr Pro Thr Ile Ala Pro Pro Pro Pro Pro Pro Gln
            420                 425                 430

cca gcg gca ccg gaa ccg gaa ctg ccc aag ccg aaa ctc aca ccc gcc    1344
Pro Ala Ala Pro Glu Pro Glu Leu Pro Lys Pro Lys Leu Thr Pro Ala
        435                 440                 445

cag gag cgg ctc aag cgg gct ata tat ata gat cgg tcc tcg cgc gtc    1392
Gln Glu Arg Leu Lys Arg Ala Ile Tyr Ile Asp Arg Ser Ser Arg Val
    450                 455                 460

ctt ttc ccc gcc ctc ttc gcc agt ctg aat ggc atc tac tgg tgt gtg    1440
Leu Phe Pro Ala Leu Phe Ala Ser Leu Asn Gly Ile Tyr Trp Cys Val
465                 470                 475                 480

ttc tac gag tat cta taa                                            1458
Phe Tyr Glu Tyr Leu
                485

<210> 12
<211> 485
<212> PRT
<213> Drosophila melanogaster

<400> 12
Met Val Phe Gln Ile Ile Ile Leu Val Ile Cys Thr Ile Cys Met Lys
1               5                   10                  15

His Tyr Ala Lys Gly Glu Phe Gln Gln Ser Leu Ala Ile Thr Asp Ile
            20                  25                  30

Leu Pro Glu Asp Ile Lys Arg Tyr Asp Lys Met Arg Pro Pro Lys Lys
        35                  40                  45

Glu Gly Gln Pro Thr Ile Val Tyr Phe His Val Thr Val Met Gly Leu
    50                  55                  60

Asp Ser Ile Asp Glu Asn Ser Met Thr Tyr Val Ala Asp Val Phe Phe
65                  70                  75                  80

Ala Gln Thr Trp Lys Asp His Arg Leu Arg Leu Pro Glu Asn Met Thr
                85                  90                  95

Gln Glu Tyr Arg Leu Leu Glu Val Asp Trp Leu Lys Asn Met Trp Arg
            100                 105                 110

Pro Asp Ser Phe Phe Lys Asn Ala Lys Ser Val Thr Phe Gln Thr Met
        115                 120                 125

Thr Ile Pro Asn His Tyr Met Trp Leu Tyr Lys Asp Lys Thr Ile Leu
    130                 135                 140

Tyr Met Val Lys Leu Thr Leu Lys Leu Ser Cys Ile Met Asn Phe Ala
145                 150                 155                 160

Ile Tyr Pro His Asp Thr Gln Glu Cys Lys Leu Gln Met Glu Ser Leu
                165                 170                 175

Ser His Thr Thr Asp Asp Leu Ile Phe Gln Trp Asp Pro Thr Thr Pro
            180                 185                 190

Leu Val Val Asp Glu Asn Ile Glu Leu Pro Gln Val Ala Leu Ile Arg
        195                 200                 205

Asn Glu Thr Ala Asp Cys Thr Gln Val Tyr Ser Thr Gly Asn Phe Thr
    210                 215                 220

Cys Leu Glu Val Val Phe Thr Leu Lys Arg Arg Leu Val Tyr Tyr Val
225                 230                 235                 240

Phe Asn Thr Tyr Ile Pro Thr Cys Met Ile Val Ile Met Ser Trp Val
                245                 250                 255

Ser Phe Trp Ile Lys Pro Glu Ala Ala Pro Ala Arg Val Thr Leu Gly
            260                 265                 270

Val Thr Ser Leu Leu Thr Leu Ser Thr Gln His Ala Lys Ser Gln Ser
        275                 280                 285

Ser Leu Pro Pro Val Ser Tyr Leu Lys Ala Val Asp Ala Phe Met Ser
    290                 295                 300

Val Cys Thr Val Phe Val Phe Met Ala Leu Met Glu Tyr Cys Leu Ile
305                 310                 315                 320

Asn Ile Val Leu Ser Asp Thr Pro Ile Pro Lys Pro Met Ala Tyr Pro
                325                 330                 335

Pro Lys Pro Val Ala Gly Asp Gly Pro Lys Lys Glu Gly Glu Gly Ala
            340                 345                 350

Pro Pro Gly Gly Ser Asn Ser Thr Ala Ser Lys Gln Gln Ala Thr Met
        355                 360                 365

Leu Pro Leu Ala Asp Glu Lys Ile Glu Lys Ile Glu Lys Ile Phe Asp
    370                 375                 380

Glu Met Thr Lys Asn Arg Arg Ile Val Thr Thr Thr Arg Arg Val Val
385                 390                 395                 400

Arg Pro Pro Leu Asp Ala Asp Gly Pro Trp Ile Pro Arg Gln Glu Ser
                405                 410                 415

Arg Ile Ile Leu Thr Pro Thr Ile Ala Pro Pro Pro Pro Pro Pro Gln
            420                 425                 430

Pro Ala Ala Pro Glu Pro Glu Leu Pro Lys Pro Lys Leu Thr Pro Ala
        435                 440                 445

Gln Glu Arg Leu Lys Arg Ala Ile Tyr Ile Asp Arg Ser Ser Arg Val
    450                 455                 460

Leu Phe Pro Ala Leu Phe Ala Ser Leu Asn Gly Ile Tyr Trp Cys Val
465                 470                 475                 480

Phe Tyr Glu Tyr Leu
                485

<210> 13
<211> 1491
<212> DNA
<213> Drosophila melanogaster

<220>
<221> CDS
<222> (1)..(1488)

<400> 13
atg gcc aat gga tcg gtg gtg gtg tcg cct ttg aac acc acg gat gct    48
Met Ala Asn Gly Ser Val Val Val Ser Pro Leu Asn Thr Thr Asp Ala
  1               5                  10                  15

ttc agt gtg tcc atc aac ctg tcg caa tcg act gtg aac aat tgt cct    96
Phe Ser Val Ser Ile Asn Leu Ser Gln Ser Thr Val Asn Asn Cys Pro
             20                  25                  30

tcg ctg aaa aac gca gag tcc atg gcg ctg atg gag ctg ctg acg aga    144
Ser Leu Lys Asn Ala Glu Ser Met Ala Leu Met Glu Leu Leu Thr Arg
         35                  40                  45

cta acc gcc ccc tgt cgc tat gac agg atg gtt cca ccc gtg gtc cac 192
Leu Thr Ala Pro Cys Arg Tyr Asp Arg Met Val Pro Pro Val Val His
    50                  55                  60

aac aaa gat ggt gag gag gta ccc atg gac ata tat gcc cgg ttt tac 240
Asn Lys Asp Gly Glu Glu Val Pro Met Asp Ile Tyr Ala Arg Phe Tyr
65                  70                  75                  80

ata tat gtg atg aag aac ctg gac tcg agt gac ctg cag ttt acg gtg 288
Ile Tyr Val Met Lys Asn Leu Asp Ser Ser Asp Leu Gln Phe Thr Val
                85                  90                  95

cag ggt ctg ctg cag ttg cga tac ctg gac ccg cgc ctg gcc ttc tcc 336
Gln Gly Leu Leu Gln Leu Arg Tyr Leu Asp Pro Arg Leu Ala Phe Ser
            100                 105                 110

agc tat ctg cct aac cga agg cag ccc ata atg ggg gag tcg gaa ctg 384
Ser Tyr Leu Pro Asn Arg Arg Gln Pro Ile Met Gly Glu Ser Glu Leu
        115                 120                 125

aag aag atg ctc tgg gtg cca cat ata ttc ctg acc aac gaa cag gcc 432
Lys Lys Met Leu Trp Val Pro His Ile Phe Leu Thr Asn Glu Gln Ala
    130                 135                 140

tcc act gtc ctg ggc acc agc gcc aag gac gag ctg acc agc att tat 480
Ser Thr Val Leu Gly Thr Ser Ala Lys Asp Glu Leu Thr Ser Ile Tyr
145                 150                 155                 160

ccg aat ggc aca gtt ctt aca tcc acg cgg ctg cag gcc aca ctg tac 528
Pro Asn Gly Thr Val Leu Thr Ser Thr Arg Leu Gln Ala Thr Leu Tyr
                165                 170                 175

tgc tgg atg aac ttc cag aag ttt ccg ttt gat gag cag aag tgt aag 576
Cys Trp Met Asn Phe Gln Lys Phe Pro Phe Asp Glu Gln Lys Cys Lys
            180                 185                 190

acc aca ttg gaa agt tgg atg tac aac acc acg ctg gtg caa ctc cac 624
Thr Thr Leu Glu Ser Trp Met Tyr Asn Thr Thr Leu Val Gln Leu His
        195                 200                 205

tgg gag acc gat aac cca gtg agt ttc gac aag caa ctt cag ctg acg 672
Trp Glu Thr Asp Asn Pro Val Ser Phe Asp Lys Gln Leu Gln Leu Thr
    210                 215                 220

gaa tac aac cta att ggg tcg ctg tac aac gaa tcc atc cga gtg tcc 720
Glu Tyr Asn Leu Ile Gly Ser Leu Tyr Asn Glu Ser Ile Arg Val Ser
225                 230                 235                 240

aac gaa tcc tac atg tcc cat ggc tcc ttg gag ggc aac tac agc atc 768
Asn Glu Ser Tyr Met Ser His Gly Ser Leu Glu Gly Asn Tyr Ser Ile
                245                 250                 255

att agc ttc acc gtt ctg ctt act cgg gaa gtg ggc tac tac gtg atc 816
Ile Ser Phe Thr Val Leu Leu Thr Arg Glu Val Gly Tyr Tyr Val Ile
            260                 265                 270

gac tac ttt ctg ccc tcg atc atg ata gtc acc att tcg tgg gtg tcc 864
Asp Tyr Phe Leu Pro Ser Ile Met Ile Val Thr Ile Ser Trp Val Ser
        275                 280                 285

ttc tgg ctg cag gcg gat caa acc ccc gca agg act act ctg ggc tgc 912
Phe Trp Leu Gln Ala Asp Gln Thr Pro Ala Arg Thr Thr Leu Gly Cys
    290                 295                 300

acc acg ctc cta tcc ttc atc act ctg tcc ttg tcc cag gag aac aat 960
Thr Thr Leu Leu Ser Phe Ile Thr Leu Ser Leu Ser Gln Glu Asn Asn
305                 310                 315                 320

ctg atg aag gtg agc tac gtg acc atg tcg gag gtg tgg ttc ctg gtc 1008
Leu Met Lys Val Ser Tyr Val Thr Met Ser Glu Val Trp Phe Leu Val
                325                 330                 335

tgc acc atc ttc att ttc ggc agc ctg gtg gag ttc gcc ttt gtc aac 1056
Cys Thr Ile Phe Ile Phe Gly Ser Leu Val Glu Phe Ala Phe Val Asn
            340                 345                 350

acc atc tgg agg cga aac aac gat ctg caa ctc aag aag cga acc acc 1104
Thr Ile Trp Arg Arg Asn Asn Asp Leu Gln Leu Lys Lys Arg Thr Thr
        355                 360                 365

aag tat ata gtc aag tcc aca ttt gtc cca cat ctg aag aaa cat cgg 1152
Lys Tyr Ile Val Lys Ser Thr Phe Val Pro His Leu Lys Lys His Arg
    370                 375                 380

cgg cat gga tat aga agg acc gat agc act atg agc acc atg agc acc 1200
Arg His Gly Tyr Arg Arg Thr Asp Ser Thr Met Ser Thr Met Ser Thr
385                 390                 395                 400

acc agc atg gac aag acc tgc ggt ccc aac aac acc gtg atc acc ata 1248
Thr Ser Met Asp Lys Thr Cys Gly Pro Asn Asn Thr Val Ile Thr Ile
                405                 410                 415

gag aca cca att att att gga ggc agt ctt agt cgc gag gac tcg gcc 1296
Glu Thr Pro Ile Ile Ile Gly Gly Ser Leu Ser Arg Glu Asp Ser Ala
            420                 425                 430

att agc ttg gac gag cag gac gag acc tcc acc tcg gag tcc agc gat    1344
Ile Ser Leu Asp Glu Gln Asp Glu Thr Ser Thr Ser Glu Ser Ser Asp
        435                 440                 445

tcg tcg aag gag aaa ccc gcg cag acc ttc gcc acc atg aca ccc aag    1392
Ser Ser Lys Glu Lys Pro Ala Gln Thr Phe Ala Thr Met Thr Pro Lys
    450                 455                 460

gag gtg tcc ctg tgg atc gac cgc aag atg cgg ttc gtc ttc cct ctg    1440
Glu Val Ser Leu Trp Ile Asp Arg Lys Met Arg Phe Val Phe Pro Leu
465                 470                 475                 480

tcc ttc atc gtg ttc aac gcc ttg ttt tgg acc ctg gtc tac tgc ctt    1488
Ser Phe Ile Val Phe Asn Ala Leu Phe Trp Thr Leu Val Tyr Cys Leu
                485                 490                 495

tga                                                                1491

<210> 14
<211> 496
<212> PRT
<213> Drosophila melanogaster

<400> 14
Met Ala Asn Gly Ser Val Val Val Ser Pro Leu Asn Thr Thr Asp Ala
  1               5                  10                  15

Phe Ser Val Ser Ile Asn Leu Ser Gln Ser Thr Val Asn Asn Cys Pro
             20                  25                  30

Ser Leu Lys Asn Ala Glu Ser Met Ala Leu Met Glu Leu Leu Thr Arg
         35                  40                  45

Leu Thr Ala Pro Cys Arg Tyr Asp Arg Met Val Pro Pro Val Val His
     50                  55                  60

Asn Lys Asp Gly Glu Glu Val Pro Met Asp Ile Tyr Ala Arg Phe Tyr
 65                  70                  75                  80

Ile Tyr Val Met Lys Asn Leu Asp Ser Ser Asp Leu Gln Phe Thr Val
                 85                  90                  95

Gln Gly Leu Leu Gln Leu Arg Tyr Leu Asp Pro Arg Leu Ala Phe Ser
            100                 105                 110

Ser Tyr Leu Pro Asn Arg Arg Gln Pro Ile Met Gly Glu Ser Glu Leu

115                 120                 125

Lys Lys Met Leu Trp Val Pro His Ile Phe Leu Thr Asn Glu Gln Ala
    130                 135                 140

Ser Thr Val Leu Gly Thr Ser Ala Lys Asp Glu Leu Thr Ser Ile Tyr
145                 150                 155                 160

Pro Asn Gly Thr Val Leu Thr Ser Thr Arg Leu Gln Ala Thr Leu Tyr
                165                 170                 175

Cys Trp Met Asn Phe Gln Lys Phe Pro Phe Asp Glu Gln Lys Cys Lys
            180                 185                 190

Thr Thr Leu Glu Ser Trp Met Tyr Asn Thr Thr Leu Val Gln Leu His
        195                 200                 205

Trp Glu Thr Asp Asn Pro Val Ser Phe Asp Lys Gln Leu Gln Leu Thr
    210                 215                 220

Glu Tyr Asn Leu Ile Gly Ser Leu Tyr Asn Glu Ser Ile Arg Val Ser
225                 230                 235                 240

Asn Glu Ser Tyr Met Ser His Gly Ser Leu Glu Gly Asn Tyr Ser Ile
                245                 250                 255

Ile Ser Phe Thr Val Leu Leu Thr Arg Glu Val Gly Tyr Tyr Val Ile
            260                 265                 270

Asp Tyr Phe Leu Pro Ser Ile Met Ile Val Thr Ile Ser Trp Val Ser
        275                 280                 285

Phe Trp Leu Gln Ala Asp Gln Thr Pro Ala Arg Thr Thr Leu Gly Cys
    290                 295                 300

Thr Thr Leu Leu Ser Phe Ile Thr Leu Ser Leu Ser Gln Glu Asn Asn
305                 310                 315                 320

Leu Met Lys Val Ser Tyr Val Thr Met Ser Glu Val Trp Phe Leu Val
                325                 330                 335

Cys Thr Ile Phe Ile Phe Gly Ser Leu Val Glu Phe Ala Phe Val Asn
            340                 345                 350

Thr Ile Trp Arg Arg Asn Asn Asp Leu Gln Leu Lys Lys Arg Thr Thr
        355                 360                 365

Lys Tyr Ile Val Lys Ser Thr Phe Val Pro His Leu Lys Lys His Arg

370                 375                 380

Arg His Gly Tyr Arg Arg Thr Asp Ser Thr Met Ser Thr Met Ser Thr
385                 390                 395                 400

Thr Ser Met Asp Lys Thr Cys Gly Pro Asn Asn Thr Val Ile Thr Ile
                405                 410                 415

Glu Thr Pro Ile Ile Ile Gly Gly Ser Leu Ser Arg Glu Asp Ser Ala
            420                 425                 430

Ile Ser Leu Asp Glu Gln Asp Glu Thr Ser Thr Ser Glu Ser Ser Asp
        435                 440                 445

Ser Ser Lys Glu Lys Pro Ala Gln Thr Phe Ala Thr Met Thr Pro Lys
    450                 455                 460

Glu Val Ser Leu Trp Ile Asp Arg Lys Met Arg Phe Val Phe Pro Leu
465                 470                 475                 480

Ser Phe Ile Val Phe Asn Ala Leu Phe Trp Thr Leu Val Tyr Cys Leu
                485                 490                 495

<210> 15
<211> 1353
<212> DNA
<213> Drosophila melanogaster

<220>
<221> CDS
<222> (1)..(1350)

<400> 15
atg ttt ctg ttc gca gaa tac gac tct gat gga aaa cga ctg ccc att      48
Met Phe Leu Phe Ala Glu Tyr Asp Ser Asp Gly Lys Arg Leu Pro Ile
1               5                   10                  15

gtg gtg aag acg cgt att tat gtg tac ttc ctg cag aac ctc aac tcc      96
Val Val Lys Thr Arg Ile Tyr Val Tyr Phe Leu Gln Asn Leu Asn Ser
            20                  25                  30

gac ctg ctg cag ttc aag atg cac gcg ctg ttg cag ctg cga ttt cag      144
Asp Leu Leu Gln Phe Lys Met His Ala Leu Leu Gln Leu Arg Phe Gln
        35                  40                  45

gac aag cgg ctg gct tac aag gcc ttc aat cgc agc gac aac atc ctg      192

Asp Lys Arg Leu Ala Tyr Lys Ala Phe Asn Arg Ser Asp Asn Ile Leu
    50                  55                  60

ggc cag aag cac ctg agc gaa cgc ctc tgg ctg ccg cac atc ttc ttt    240
Gly Gln Lys His Leu Ser Glu Arg Leu Trp Leu Pro His Ile Phe Phe
 65                  70                  75                  80

gcc aac gaa cga gag tcc agt atc ctg ggc acg gac gag aag gac gtc    288
Ala Asn Glu Arg Glu Ser Ser Ile Leu Gly Thr Asp Glu Lys Asp Val
                 85                  90                  95

ctg act tcg ctt tcg ccc gag gga aac gtg atc atc tcc acg cgc atg    336
Leu Thr Ser Leu Ser Pro Glu Gly Asn Val Ile Ile Ser Thr Arg Met
            100                 105                 110

cag gcc agt ctc tac tgc tgg atg aac ttt cag aag ttt ccc ttt gac    384
Gln Ala Ser Leu Tyr Cys Trp Met Asn Phe Gln Lys Phe Pro Phe Asp
        115                 120                 125

cag cag ttc tgc tcc acg gtc ctg gag agc tgg atg tac aac act tcc    432
Gln Gln Phe Cys Ser Thr Val Leu Glu Ser Trp Met Tyr Asn Thr Ser
    130                 135                 140

gat ttg att ctg gag tgg gag ccg cac acg ccc atc tcc ttt gac ccg    480
Asp Leu Ile Leu Glu Trp Glu Pro His Thr Pro Ile Ser Phe Asp Pro
145                 150                 155                 160

gaa atg agg ctc act gag tac aat atg gcc cag ttc tgg cac aac act    528
Glu Met Arg Leu Thr Glu Tyr Asn Met Ala Gln Phe Trp His Asn Thr
                165                 170                 175

acg att gtg cag tcg gat ggg gac aat ctg cgg cat gga gct ttt gct    576
Thr Ile Val Gln Ser Asp Gly Asp Asn Leu Arg His Gly Ala Phe Ala
            180                 185                 190

ggt aac tat agt tcg ctg agc ttt acg gtc aat ctg aag cga gaa att    624
Gly Asn Tyr Ser Ser Leu Ser Phe Thr Val Asn Leu Lys Arg Glu Ile
        195                 200                 205

ggc ttc tat ttg ctc gac tat tac ctg ccc tcc atg atg atc gtg gcc    672
Gly Phe Tyr Leu Leu Asp Tyr Tyr Leu Pro Ser Met Met Ile Val Ala
    210                 215                 220

atc tct tgg gtg tcc ttc tgg ctt cag gcg gac gca tct cct cca agg    720
Ile Ser Trp Val Ser Phe Trp Leu Gln Ala Asp Ala Ser Pro Pro Arg
225                 230                 235                 240

att atg ctg gga acc agc acc atg ttg tcg ttc atc acg ctt tcc tca    768

Ile Met Leu Gly Thr Ser Thr Met Leu Ser Phe Ile Thr Leu Ser Ser
                245                 250                 255

tcg cag agc aag aac ctg ccc aag gtg agc tac ata aag gtg tcg gag     816
Ser Gln Ser Lys Asn Leu Pro Lys Val Ser Tyr Ile Lys Val Ser Glu
            260                 265                 270

gtg tgg tgc ctg ggc tgc acc ttc ttc atc ttc ggc agc ctg gtg gag     864
Val Trp Cys Leu Gly Cys Thr Phe Phe Ile Phe Gly Ser Leu Val Glu
        275                 280                 285

ttt gcc ttt gtc aac acc att tgg cgc cgc aag gag aac att gag ctg     912
Phe Ala Phe Val Asn Thr Ile Trp Arg Arg Lys Glu Asn Ile Glu Leu
    290                 295                 300

aag aag gtc aac agc aag tac atc ata aaa tcc aca ttg aca cca cga     960
Lys Lys Val Asn Ser Lys Tyr Ile Ile Lys Ser Thr Leu Thr Pro Arg
305                 310                 315                 320

ccg gcc cgc cgt caa att ggc gga agt ttg agc aac gag tcc agg gct    1008
Pro Ala Arg Arg Gln Ile Gly Gly Ser Leu Ser Asn Glu Ser Arg Ala
                325                 330                 335

aga tcg tgt tcc agt ttg gac aac att gtt tcc agc acg gag agc gtg    1056
Arg Ser Cys Ser Ser Leu Asp Asn Ile Val Ser Ser Thr Glu Ser Val
            340                 345                 350

cgc aat ggc aat ggc act gtg aat cag ggc ttc aac aac tat ttg aca    1104
Arg Asn Gly Asn Gly Thr Val Asn Gln Gly Phe Asn Asn Tyr Leu Thr
        355                 360                 365

gtg cat ccc aat tta ccc atc ata aga acg gag tgc gcc gag gca gat    1152
Val His Pro Asn Leu Pro Ile Ile Arg Thr Glu Cys Ala Glu Ala Asp
    370                 375                 380

aca gtg tct atc tgc agt gcg cgt acg aac aac gat cat atc gtg gat    1200
Thr Val Ser Ile Cys Ser Ala Arg Thr Asn Asn Asp His Ile Val Asp
385                 390                 395                 400

gtg gac aag gat aaa aag gac acg ccg ccc acc ttt acc acc atg aca    1248
Val Asp Lys Asp Lys Lys Asp Thr Pro Pro Thr Phe Thr Thr Met Thr
                405                 410                 415

ccg cag gag atc gcc atg tgg ata gac cgg cgg tcg cga ttt ctt ttt    1296
Pro Gln Glu Ile Ala Met Trp Ile Asp Arg Arg Ser Arg Phe Leu Phe
            420                 425                 430

ccg gcc atg ttc ctc gcc ttc aac gct ctg tat tgg acc ttc gtc tat    1344

Pro Ala Met Phe Leu Ala Phe Asn Ala Leu Tyr Trp Thr Phe Val Tyr
        435                 440                 445

gtt ttg taa 1353
Val Leu
    450

<210> 16
<211> 450
<212> PRT
<213> Drosophila melanogaster

<400> 16
Met Phe Leu Phe Ala Glu Tyr Asp Ser Asp Gly Lys Arg Leu Pro Ile
1               5                   10                  15

Val Val Lys Thr Arg Ile Tyr Val Tyr Phe Leu Gln Asn Leu Asn Ser
            20                  25                  30

Asp Leu Leu Gln Phe Lys Met His Ala Leu Leu Gln Leu Arg Phe Gln
        35                  40                  45

Asp Lys Arg Leu Ala Tyr Lys Ala Phe Asn Arg Ser Asp Asn Ile Leu
    50                  55                  60

Gly Gln Lys His Leu Ser Glu Arg Leu Trp Leu Pro His Ile Phe Phe
65                  70                  75                  80

Ala Asn Glu Arg Glu Ser Ser Ile Leu Gly Thr Asp Glu Lys Asp Val
                85                  90                  95

Leu Thr Ser Leu Ser Pro Glu Gly Asn Val Ile Ile Ser Thr Arg Met
            100                 105                 110

Gln Ala Ser Leu Tyr Cys Trp Met Asn Phe Gln Lys Phe Pro Phe Asp
        115                 120                 125

Gln Gln Phe Cys Ser Thr Val Leu Glu Ser Trp Met Tyr Asn Thr Ser
    130                 135                 140

Asp Leu Ile Leu Glu Trp Glu Pro His Thr Pro Ile Ser Phe Asp Pro
145                 150                 155                 160

Glu Met Arg Leu Thr Glu Tyr Asn Met Ala Gln Phe Trp His Asn Thr
                165                 170                 175

Thr Ile Val Gln Ser Asp Gly Asp Asn Leu Arg His Gly Ala Phe Ala

            180                 185                 190

Gly Asn Tyr Ser Ser Leu Ser Phe Thr Val Asn Leu Lys Arg Glu Ile
        195                 200                 205

Gly Phe Tyr Leu Leu Asp Tyr Tyr Leu Pro Ser Met Met Ile Val Ala
    210                 215                 220

Ile Ser Trp Val Ser Phe Trp Leu Gln Ala Asp Ala Ser Pro Pro Arg
225                 230                 235                 240

Ile Met Leu Gly Thr Ser Thr Met Leu Ser Phe Ile Thr Leu Ser Ser
                245                 250                 255

Ser Gln Ser Lys Asn Leu Pro Lys Val Ser Tyr Ile Lys Val Ser Glu
            260                 265                 270

Val Trp Cys Leu Gly Cys Thr Phe Phe Ile Phe Gly Ser Leu Val Glu
        275                 280                 285

Phe Ala Phe Val Asn Thr Ile Trp Arg Arg Lys Glu Asn Ile Glu Leu
    290                 295                 300

Lys Lys Val Asn Ser Lys Tyr Ile Ile Lys Ser Thr Leu Thr Pro Arg
305                 310                 315                 320

Pro Ala Arg Arg Gln Ile Gly Gly Ser Leu Ser Asn Glu Ser Arg Ala
                325                 330                 335

Arg Ser Cys Ser Ser Leu Asp Asn Ile Val Ser Ser Thr Glu Ser Val
            340                 345                 350

Arg Asn Gly Asn Gly Thr Val Asn Gln Gly Phe Asn Asn Tyr Leu Thr
        355                 360                 365

Val His Pro Asn Leu Pro Ile Ile Arg Thr Glu Cys Ala Glu Ala Asp
    370                 375                 380

Thr Val Ser Ile Cys Ser Ala Arg Thr Asn Asn Asp His Ile Val Asp
385                 390                 395                 400

Val Asp Lys Asp Lys Lys Asp Thr Pro Pro Thr Phe Thr Thr Met Thr
                405                 410                 415

Pro Gln Glu Ile Ala Met Trp Ile Asp Arg Arg Ser Arg Phe Leu Phe
            420                 425                 430

Pro Ala Met Phe Leu Ala Phe Asn Ala Leu Tyr Trp Thr Phe Val Tyr

435                 440                 445

Val Leu
    450

<210> 17
<211> 1257
<212> DNA
<213> Drosophila melanogaster

<220>
<221> CDS
<222> (1)..(1254)

<400> 17
atg ctg agc gaa gcc acg gaa gcc cac atg aac tac aca gcc aag ccg      48
Met Leu Ser Glu Ala Thr Glu Ala His Met Asn Tyr Thr Ala Lys Pro
1               5                   10                  15

cga gta gtt ctt ccg cct aat tat gtc aag gaa ata cga ccg ccc tcg      96
Arg Val Val Leu Pro Pro Asn Tyr Val Lys Glu Ile Arg Pro Pro Ser
            20                  25                  30

aag aag ggc tca cca gtg ata gta gac ttt agc ata ttc gtt gta gat     144
Lys Lys Gly Ser Pro Val Ile Val Asp Phe Ser Ile Phe Val Val Asp
        35                  40                  45

att aac tca att aac gta gag gat atg gac ttt aga gta gac atg ttt     192
Ile Asn Ser Ile Asn Val Glu Asp Met Asp Phe Arg Val Asp Met Phe
    50                  55                  60

ata cat cag cgc tgg ctg gag tcg cgt ctg gag atc cca gat gac ata     240
Ile His Gln Arg Trp Leu Glu Ser Arg Leu Glu Ile Pro Asp Asp Ile
65                  70                  75                  80

ttc gag gag ggc gac gac tac gtg acg ctg ctt cca gag gtc ttt gag     288
Phe Glu Glu Gly Asp Asp Tyr Val Thr Leu Leu Pro Glu Val Phe Glu
                85                  90                  95

aat ttt tgg cag ccg gat ccg tac ttt ctt aat tcc aaa att gcc gaa     336
Asn Phe Trp Gln Pro Asp Pro Tyr Phe Leu Asn Ser Lys Ile Ala Glu
            100                 105                 110

ata gcg aca ctg acc cac aag ttc acg tcg gtg acg ctc tac aag aac     384
Ile Ala Thr Leu Thr His Lys Phe Thr Ser Val Thr Leu Tyr Lys Asn
        115                 120                 125

aaa acg gtg cgc tat gcg gcc cgg atg cat gcg atc atc gcc tgc cag 432
Lys Thr Val Arg Tyr Ala Ala Arg Met His Ala Ile Ile Ala Cys Gln
    130                 135                 140

atg gag ttc cag ctg tac ccc atg gac atc cag gtg tgt ccc atc tac 480
Met Glu Phe Gln Leu Tyr Pro Met Asp Ile Gln Val Cys Pro Ile Tyr
145                 150                 155                 160

atc gag agc ttc tcg tcg aac aat caa aag gtt aag ctg cgc tgg tcc 528
Ile Glu Ser Phe Ser Ser Asn Asn Gln Lys Val Lys Leu Arg Trp Ser
                165                 170                 175

gac tcc ggg gtg acc ctc aat ccg gaa ctg aag ctg ctg cag tac aat 576
Asp Ser Gly Val Thr Leu Asn Pro Glu Leu Lys Leu Leu Gln Tyr Asn
            180                 185                 190

ctg ggc cag ccg ctg gaa ctg gag gag agc gac ggc tac atg ccg gag 624
Leu Gly Gln Pro Leu Glu Leu Glu Glu Ser Asp Gly Tyr Met Pro Glu
        195                 200                 205

aag gtg ggg aac ttc tcc cgc ctc acc gta tac ttc cgc ttc gag cgc 672
Lys Val Gly Asn Phe Ser Arg Leu Thr Val Tyr Phe Arg Phe Glu Arg
    210                 215                 220

cag att ggc cac cac ctc atc cag acc ttc gcc ccc tca tcg ctg gtg 720
Gln Ile Gly His His Leu Ile Gln Thr Phe Ala Pro Ser Ser Leu Val
225                 230                 235                 240

ctt ctg gct ggg act gga cgc cat tcc ggg gcg cgt aac gct act ggt 768
Leu Leu Ala Gly Thr Gly Arg His Ser Gly Ala Arg Asn Ala Thr Gly
                245                 250                 255

gac ctg cat gct gac cct ggt gac cat gtt acg ggc gcc gac att ccg 816
Asp Leu His Ala Asp Pro Gly Asp His Val Thr Gly Ala Asp Ile Pro
            260                 265                 270

cca gtg gcc tac gtt aag gca ctg gat ctc tgg atg gcc ggg tgc atg 864
Pro Val Ala Tyr Val Lys Ala Leu Asp Leu Trp Met Ala Gly Cys Met
        275                 280                 285

ctg tcc gtg ttc gcc gcg ctg gcc gag ttc gtg gtg gtg aaa gtg ctg 912
Leu Ser Val Phe Ala Ala Leu Ala Glu Phe Val Val Val Lys Val Leu
    290                 295                 300

gac gtg cag tac cag tac cag gtg aac cgc ata ccc aag gta ctg ccc 960
Asp Val Gln Tyr Gln Tyr Gln Val Asn Arg Ile Pro Lys Val Leu Pro
305                 310                 315                 320

atg cgc atc agc aat atg gag aag ggt cag tgt gcg acg gtg gcc agt    1008
Met Arg Ile Ser Asn Met Glu Lys Gly Gln Cys Ala Thr Val Ala Ser
                325                 330                 335

tgg gag ggc gga gcg gtg cgg tcg cgg aag gcc act cag acg ccc acg    1056
Trp Glu Gly Gly Ala Val Arg Ser Arg Lys Ala Thr Gln Thr Pro Thr
            340                 345                 350

aca ccg ggc cag ggt aac ggc gga ccg ccg aag cca gca aga cgt caa    1104
Thr Pro Gly Gln Gly Asn Gly Gly Pro Pro Lys Pro Ala Arg Arg Gln
        355                 360                 365

agt cta ctt tcc gtg gcg tgg acg gat acg gat acc ggc gtg gag aag    1152
Ser Leu Leu Ser Val Ala Trp Thr Asp Thr Asp Thr Gly Val Glu Lys
    370                 375                 380

atc atg tgg cga gag atc gac aag gtg tcc cgc gcc gta ttc ccc atc    1200
Ile Met Trp Arg Glu Ile Asp Lys Val Ser Arg Ala Val Phe Pro Ile
385                 390                 395                 400

ctg ttc ttt gtg ttt gtg ctc ctg tac tgg ccg ata ctg ctg atg aag    1248
Leu Phe Phe Val Phe Val Leu Leu Tyr Trp Pro Ile Leu Leu Met Lys
                405                 410                 415

tcg tcc tag                                                        1257
Ser Ser

<210> 18
<211> 418
<212> PRT
<213> Drosophila melanogaster

<400> 18
Met Leu Ser Glu Ala Thr Glu Ala His Met Asn Tyr Thr Ala Lys Pro
1               5                   10                  15

Arg Val Val Leu Pro Pro Asn Tyr Val Lys Glu Ile Arg Pro Pro Ser
            20                  25                  30

Lys Lys Gly Ser Pro Val Ile Val Asp Phe Ser Ile Phe Val Val Asp
        35                  40                  45

Ile Asn Ser Ile Asn Val Glu Asp Met Asp Phe Arg Val Asp Met Phe
    50                  55                  60

Ile His Gln Arg Trp Leu Glu Ser Arg Leu Glu Ile Pro Asp Asp Ile

65                  70                  75                  80

Phe Glu Glu Gly Asp Asp Tyr Val Thr Leu Leu Pro Glu Val Phe Glu
                85                  90                  95

Asn Phe Trp Gln Pro Asp Pro Tyr Phe Leu Asn Ser Lys Ile Ala Glu
            100                 105                 110

Ile Ala Thr Leu Thr His Lys Phe Thr Ser Val Thr Leu Tyr Lys Asn
        115                 120                 125

Lys Thr Val Arg Tyr Ala Ala Arg Met His Ala Ile Ile Ala Cys Gln
    130                 135                 140

Met Glu Phe Gln Leu Tyr Pro Met Asp Ile Gln Val Cys Pro Ile Tyr
145                 150                 155                 160

Ile Glu Ser Phe Ser Ser Asn Asn Gln Lys Val Lys Leu Arg Trp Ser
                165                 170                 175

Asp Ser Gly Val Thr Leu Asn Pro Glu Leu Lys Leu Leu Gln Tyr Asn
            180                 185                 190

Leu Gly Gln Pro Leu Glu Leu Glu Glu Ser Asp Gly Tyr Met Pro Glu
        195                 200                 205

Lys Val Gly Asn Phe Ser Arg Leu Thr Val Tyr Phe Arg Phe Glu Arg
    210                 215                 220

Gln Ile Gly His His Leu Ile Gln Thr Phe Ala Pro Ser Ser Leu Val
225                 230                 235                 240

Leu Leu Ala Gly Thr Gly Arg His Ser Gly Ala Arg Asn Ala Thr Gly
                245                 250                 255

Asp Leu His Ala Asp Pro Gly Asp His Val Thr Gly Ala Asp Ile Pro
            260                 265                 270

Pro Val Ala Tyr Val Lys Ala Leu Asp Leu Trp Met Ala Gly Cys Met
        275                 280                 285

Leu Ser Val Phe Ala Ala Leu Ala Glu Phe Val Val Val Lys Val Leu
    290                 295                 300

Asp Val Gln Tyr Gln Tyr Gln Val Asn Arg Ile Pro Lys Val Leu Pro
305                 310                 315                 320

Met Arg Ile Ser Asn Met Glu Lys Gly Gln Cys Ala Thr Val Ala Ser

                325                 330                 335

Trp Glu Gly Gly Ala Val Arg Ser Arg Lys Ala Thr Gln Thr Pro Thr
            340                 345                 350

Thr Pro Gly Gln Gly Asn Gly Gly Pro Pro Lys Pro Ala Arg Arg Gln
        355                 360                 365

Ser Leu Leu Ser Val Ala Trp Thr Asp Thr Asp Thr Gly Val Glu Lys
    370                 375                 380

Ile Met Trp Arg Glu Ile Asp Lys Val Ser Arg Ala Val Phe Pro Ile
385                 390                 395                 400

Leu Phe Phe Val Phe Val Leu Leu Tyr Trp Pro Ile Leu Leu Met Lys
                405                 410                 415

Ser Ser